(19) Europäisches Patentamt
European Patent Office
Office européen des brevets

(11) **EP 4 144 315 B1**

(12) **EUROPEAN PATENT SPECIFICATION**

(45) Date of publication and mention
of the grant of the patent:
**31.01.2024 Bulletin 2024/05**

(21) Application number: **22201421.9**

(22) Date of filing: **03.12.2020**

(51) International Patent Classification (IPC):
***A61B 18/14*** *(2006.01)*

(52) Cooperative Patent Classification (CPC):
**A61B 18/1445; A61B 18/1815; A61N 7/02;**
A61B 2018/0013; A61B 2018/00196;
A61B 2018/00202; A61B 2018/00345;
A61B 2018/00577; A61B 2018/00601;
A61B 2018/00607; A61B 2018/00619;
A61B 2018/0063; A61B 2018/00785;
A61B 2018/00797; A61B 2018/00821;     (Cont.)

(54) **ELECTROSURGICAL INSTRUMENT, GENERATOR AND APPARATUS**

ELEKTROCHIRURGISCHES INSTRUMENT, GENERATOR UND GERÄT

INSTRUMENT ÉLECTROCHIRURGICAL, GÉNÉRATEUR ET APPAREIL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.12.2019 GB 201917752**

(43) Date of publication of application:
**08.03.2023 Bulletin 2023/10**

(62) Document number(s) of the earlier application(s) in
accordance with Art. 76 EPC:
**20819732.7 / 4 069 121**

(73) Proprietor: **Creo Medical Limited
Chepstow, Monmouthshire NP16 5UH (GB)**

(72) Inventor: **HANCOCK, Christopher Paul
Chepstow, NP16 5UH (GB)**

(74) Representative: **Mewburn Ellis LLP
Aurora Building
Counterslip
Bristol BS1 6BX (GB)**

(56) References cited:
**WO-A2-2017/123837       GB-A- 2 561 167
GB-A- 2 569 812          US-A1- 2018 280 084
US-A1- 2018 333 184      US-A1- 2018 333 190**

(52) Cooperative Patent Classification (CPC): (Cont.)
A61B 2018/00869; A61B 2018/00982;
A61B 2018/00994; A61B 2018/1273;
A61B 2018/1455; A61B 2018/1823

## Description

FIELD OF THE INVENTION

The invention relates to an electrosurgical instrument for delivering electromagnetic (EM) energy and ultrasonic vibrations for treating biological tissue, the electrosurgical instrument having a magnetostrictive ultrasound transducer for generating said ultrasonic vibrations. The invention further relates to an electrosurgical generator for generating the EM energy and an electrical signal for driving said magnetostrictive ultrasound transducer.

BACKGROUND TO THE INVENTION

Electrosurgical instruments and their associated generators are pervasive throughout hospital operating theatres, for use in open and laparoscopic procedures, and are also increasingly present in endoscopy suites. In endoscopic procedures the electrosurgical accessory is typically inserted through a lumen inside an endoscope. Considered against the equivalent access channel for laparoscopic surgery, such a lumen is comparatively narrow in bore and greater in length.

It is known to use radiofrequency (RF) energy to cut biological tissue. The method of cutting using RF energy operates using the principle that as an electric current passes through a tissue matrix (aided by the ionic contents of the cells and the intercellular electrolytes), the impedance to the flow of electrons across the tissue generates heat. When an RF voltage is applied to the tissue matrix, enough heat is generated within the cells to vaporise the water content of the tissue. As a result of this increasing desiccation, particularly adjacent to the RF emitting region of the instrument (referred to herein as an RF blade) which has the highest current density of the entire current path through tissue, the tissue adjacent to the cut pole of the RF blade loses direct contact with the blade. The applied voltage then appears almost entirely across this void which ionises as a result, forming a plasma, which has a very high volume resistivity compared to tissue. This differentiation is important as it focusses the applied energy to the plasma that completed the electrical circuit between the cut pole of the RF blade and the tissue. Any volatile material entering the plasma slowly enough is vaporised and the perception is therefore of a tissue dissecting plasma.

GB 2 486 343 discloses a control system for an electrosurgical apparatus which delivers both RF and microwave energy to treat biological tissue. The energy delivery profile of both RF energy and microwave energy delivered to a probe is set based on sampled voltage and current information of RF energy conveyed to the probe and sampled forward and reflected power information for the microwave energy conveyed to and from the probe.

Forceps capable of delivering heat energy into grasped biological tissue are also known. For example, it is known to deliver radiofrequency (RF) energy from a bipolar electrode arrangement in the jaws of the forceps. The RF energy may be used to seal a vessel by thermal denaturation of extracellular matrix proteins (e.g. collagen) within the vessel wall. The heat energy may also cauterise the grasped tissue and facilitate coagulation.

Such devices typically find application on the end of minimal invasive surgical laparoscopic tools but can equally find use in other clinical procedural areas such as gynaecology, endourology, gastrointestinal surgery, ENT procedures, etc. Depending on the context of use, these devices can have differing physical construction, size, scale and complexity.

Current examples of minimally invasive device that are capable of dissecting body tissue at the same time as achieving haemostasis include the LigaSure vessel sealing technology manufactured by Covidien, and the Thunderbeat platform from Olympus. The LigaSure system is a bipolar forceps arrangement in which current is delivered to seal tissue while pressure is applied. The Thunderbeat platform simultaneously delivers thermal energy generated using an ultrasonic source, and bipolar electrical energy.

US 6,585,735 describes an endoscopic bipolar forceps in which the jaws of the forceps are arranged to conduct bipolar energy through the tissue held therebetween.

EP 2 233 098 describes microwave forceps for sealing tissue in which the sealing surfaces of the jaws include one or more microwave antennas for radiating microwave energy into tissue grasped between the jaws of the forceps.

WO 2015/097472 describes electrosurgical forceps in which one or more pairs of non-resonant unbalanced lossy transmission line structure are arranged on the inner surface of a pair of jaws.

GB2569812 discloses an electrosurgical instrument for delivering radiofrequency and microwave energy to biological tissue in order to ablate the target tissue. The instrument comprises a generator which may be arranged to monitor reflected signals in order to determine an appropriate power level for delivery or which may be arranged to calculate an impedance seen at the distal end of the instrument in order to determine an optimal delivery power level.

SUMMARY OF THE INVENTION

The invention is defined by the electrosurgical generator according to claim 1 and by an electrosurgical apparatus according to claim 11 comprising said electrosurgical generator.

At its most general, the invention provides an electrosurgical instrument for delivering electromagnetic (EM) energy and ultrasound vibrations for treating biological tissue, wherein the ultrasound vibrations are generated by a magnetostrictive ultrasound transducer. The EM energy may include radiofrequency (RF) EM energy and/or microwave EM energy. The electrosurgical instrument may include a distal end assembly which delivers the EM energy into biological tissue for tissue treatment, and the transducer may generate ultrasonic vibrations around the distal end assembly for treatment of biological tissue.

In this way, a magnetostrictive transducer may be used to generate ultrasonic vibrations for tissue treatment without the need for additional mechanical amplifying means. That is, compared to other types of ultrasonic transducer (e.g. piezoelectric) a magnetostrictive transducer can generate stronger ultrasonic vibrations, meaning that further amplification means or mechanisms may not be required. Advantageously, therefore, a magnetostrictive transducer may be positioned towards a distal tip of the instrument so as to maximise delivery of ultrasonic vibrations to biological tissue surrounding the distal end assembly.

Also, the invention provides an electrosurgical generator capable of supplying an electrical signal for driving a magnetostrictive transducer to generate ultrasonic vibrations. The electrosurgical generator may comprise an electrical signal supply unit that is integrated with means for generating EM energy (e.g. microwave electromagnetic signals and/or radiofrequency electromagnetic signals) for treatment. The electrosurgical generator may be configured to deliver different types of signal (e.g. RF, microwave, electrical signal for driving a magnetostrictive ultrasound transducer) along a common feed cable. A single generator may thus be used as the source of energy of different types of treatment. This can be advantageous in terms of minimising the equipment needed in a treatment suite. For example, ultrasonic vibrations may be used to divide (e.g. cut or dissect) biological tissue, and EM energy may be used to ablate and/or coagulate biological tissue.

Also, the magnetostrictive ultrasound transducer is a current controlled device, that is, the transducer receives an oscillating current signal in order to induce an oscillating magnetic field so as to generate an oscillating vibration. When the oscillations are at an ultrasound frequency, the ultrasonic vibrations are generated. This contrasts with other types of ultrasound transducer, such as, piezoelectric ultrasound transducers, which receive an oscillating voltage signal to create an oscillating vibration due to the piezoelectric effect.

According to a first aspect of the invention, there is provided an electrosurgical instrument for delivering electromagnetic (EM) energy and ultrasonic vibrations for treating biological tissue, the electrosurgical instrument comprising: an instrument shaft arranged to convey EM energy and an electrical signal for driving an ultrasound transducer; a distal end assembly arranged at a distal end of the instrument shaft to receive the EM energy from the instrument shaft and deliver the EM energy from the distal end assembly for tissue treatment; and a magnetostrictive ultrasound transducer arranged to receive the electrical signal from the instrument shaft and generate ultrasonic vibrations around the distal end assembly for tissue treatment.

The instrument shaft may include a coaxial transmission line having an inner conductor, an outer conductor, and a dielectric material separating the inner conductor from the outer conductor, the coaxial transmission line being arranged to convey the EM energy and the electrical signal. The EM energy may include RF and/or microwave EM energy. In this example, the RF and microwave energy is carried along the instrument shaft by a common coaxial transmission line. Also, the transducer may include first and second input terminals for receiving the electrical signal from the coaxial transmission line, the first input terminal being connected to the inner conductor by a first connection means (e.g. conductor, wire, cable or track), and the second input terminal being connected to the outer conductor by a second connection means (e.g. conductor, wire, cable or track).

In other examples, RF and microwave energy may be transported along separate energy conveying structures. For example, the RF energy can be conveyed by a twisted wire pair or two insulated wire assemblies mounted in parallel, whilst the microwave energy is carried by a suitable coaxial transmission line. Also, the electrical signal for driving the magnetostrictive transducer to generate ultrasonic vibrations can be delivered in a similar manner, i.e. the electrical signal can be delivered with EM energy (e.g. via a coaxial cable) or along a separate conveying structure (e.g. a twisted wire pair or two insulated wire assemblies mounted in parallel). In any case, the transducer will be coupled to the structure conveying the electrical signal by suitable connection means (e.g. one or more conductors, wires, cables or tracks).

The magnetostrictive transducer includes a coil of conductive material wrapped around a magnetostrictive element (e.g. solenoid or rod) made from magnetostrictive material. Magnetostriction is a property of ferromagnetic materials which causes them to expand or contract (i.e. change their physical dimensions) in response to a magnetic field (H-field). This effect allows magnetostrictive materials to convert electromagnetic energy into mechanical energy. As a magnetic field is applied to the material, its molecular dipoles and magnetic field boundaries rotate to align with the field. This causes the material to strain and elongate. To effect this change in physical dimensions, an oscillating electrical signal (e.g. current signal) is applied to the coil in order to induce an oscillating magnetic field around the magnetostrictive element which, due to its magnetostrictive properties, causes a corresponding oscillating change in the physical dimensions of the magnetostrictive element (e.g. an oscillating expansion and contraction). The oscillating electrical signal (e.g. current signal) can be selected or tuned in order to cause changes in the physical dimensions of the magnetostrictive element at an ultrasonic frequency which in turn generate ultrasonic vibrations around the magnetostrictive transducer and elements to which the transducer is connected (directly) or coupled (indirectly).

Ultrasonic frequencies are understood to be those in the range of 20kHz to 5MHz. As such, the electrical signal has a frequency of between 20kHz to 5MHz.

In an embodiment, the electrical signal is a varying or oscillating current signal. The varying or oscillating current signal may have one of the following forms: sinusoidal, square, trapezoidal, ramp, exponential. In any case, the electrical signal is applied to the coil of the magnetostrictive transducer to induce a varying magnetic field around the magnetostrictive element. Due to the magnetostrictive effect, the magnetostrictive element changes its physical dimensions with the changes in magnetic field (and the changes in current). Therefore, in order to generate ultrasonic vibrations, the electrical signal includes oscillations (e.g. oscillations in current) at an ultrasound frequency. In an embodiment, the electrical signal is an oscillating current signal which varies by up to 100A between its minimum and maximum (e.g. between 0A and 100A).

In an embodiment, the transducer comprises a magnetostrictive element made from Terfenol-D. Terfenol-D is advantageous because, relative to other magnetostrictive materials (e.g. Galfenol and Alfenol), it produces a large strain (i.e. change in physical dimension; or output) for a given stress (i.e. variation in applied magnetic field; or input).

The magnetostrictive transducer may be mounted on or in the instrument shaft. For example, the transducer may be positioned towards a distal end of the instrument shaft. If the instrument shaft includes a coaxial transmission line, the transducer may be positioned at a distal end of the coaxial transmission line, and at or near to the point at which the distal end assembly attaches to the coaxial transmission line. This arrangement may be preferable where the distal end assembly is small and complex such that it would be difficult to find room to position the transducer in the distal end assembly.

Alternatively, the magnetostrictive transducer may be mounted on or in the distal end assembly. For example, the distal end assembly may be a radiating tip portion arranged to radiate an EM field for tissue treatment. This type of electrosurgical instrument maybe suitable for use in minimally invasive surgical techniques that provide, at a very small scale, a localized microwave field capable of precisely ablating tissue, for example, in the lungs. This may be done through suitable selection of geometry and material for a radiating distal tip. The radiating tip portion may also be configured to deliver RF energy. The radiating tip portion may include: a dielectric tip, a distal conductive portion of the inner conductor, which extends longitudinally into the dielectric tip, an intermediate dielectric element surrounding a proximal part of the distal conductive portion and separating the dielectric material of the coaxial transmission line from the dielectric tip, and wherein the transducer is mounted on or in the intermediate dielectric element.

Where the EM energy and electrical signal are conveyed by the coaxial transmission line, the transducer may be electrically coupled at a first terminal to the inner conductor of the coaxial cable and at a second terminal to the outer conductor of the coaxial cable. For example, small wires or tracks may be used as connectors or couplings. Additionally, the transducer may be partly or completely embedded within a volume of the intermediate dielectric element. Additionally or alternatively, the transducer may be positioned on an inner or an outer surface of the intermediate dielectric element. In another embodiment, the transducer may be mounted on a different part of the radiating tip portion, for example, on or in the dielectric tip.

In an embodiment, the dielectric tip may be formed from a second dielectric material that has a dielectric constant different to (e.g. greater than) the dielectric material of the coaxial transmission line (aka first dielectric material).

In an embodiment, the radiating tip portion is thus a coaxial-based device with a dielectric material at its distal end to produce an omnidirectional radiation pattern to create a controllable spherical zone of ablation or coagulation. The geometry of the dielectric radiator determines the shape of the electromagnetic radiation pattern and the tissue affects produced. The distal end of the device is designed to facilitate efficient microwave energy delivery into biological tissue to achieve a localized volume of ablation or coagulation. The resulting localized, thermally induced zone of ablation or coagulation occurs as a result of dielectric heating or a combination of dielectric and thermal conduction.

The effect of the dielectric tip is to reduce the wavelength of the microwave energy and the structure of the dielectric tip is modelled, using electromagnetic field analysis software to produce better impedance matching and control of the resultant ablation profile based on the small geometry constraints imposed by the dimensions of blood vessels. For example, the outer diameter of the coaxial cable and radiating tip portion may be equal to or less than 1.9 mm, preferably equal to or less than 1.5 mm or even more preferably less than 1 mm. This size enables the instrument to fit down a vessel directly or be manipulated by commercially available miniature scoping device instrument channels. This size also enables the instrument to be inserted inside of, and travel within, a blood vessel.

In order to maintain flexibility of the device, the axial length of the dielectric tip is equal to or less than 5 mm, preferably equal to or less than 2 mm. This enables the second dielectric material to be relatively rigid without adversely affecting the flexibility of the instrument, especially at its distal end. In order to shrink the length of the tip by a large enough amount, the dielectric constant of the dielectric may need to be much greater than unity, i.e. 9 or 100, where the wavelength will be shrunk by 3 and 10 respectively,

The microwave energy may be a single spot frequency, e.g. 5.8 GHz or it may be a spot frequency that can be increased or decreased around the spot frequency, e.g.

5.8GHz +/- 100MHz or 2.45GHz +/- 50MHz. This frequency variation can be translated into a change in phase that helps tune or match the microwave energy in the tissue load.

The dielectric constant of the second dielectric material may be selected based on the frequency of the microwave energy such that the axial length of the dielectric tip corresponds to a non-negligible fraction of a wavelength of the microwave energy when propagating in the dielectric tip. Herein, a non-negligible fraction may be equal to or greater than 0.05, preferably more than 0.06. This can ensure that the second dielectric material provides a suitable wavelength-shortening effect. In one embodiment, the dielectric constant of the second dielectric material is equal to or greater than 80. For example, titanium dioxide may be used as the second dielectric material. PFTE or any other dielectric that is low loss at the frequency of the microwave energy may be used for the first dielectric material.

The radiating tip portion may be arranged to act as an impedance transformer, for example a quarter wave impedance transformer to match the effective impedance of the antenna to a tissue load impedance. In other words, the geometry of the radiating tip portion is selected so that the effects of the impedance mismatch are invisible when looking into the transmission line prior to the impedance transformer. This may also be considered as being an impedance matching network.

The radiating tip portion includes an intermediate dielectric element surrounding a proximal part of the distal conductive portion and separating the first dielectric material from the dielectric tip. The intermediate dielectric element may be formed from a third dielectric material that is different from the second dielectric material. The third dielectric material may be the same as or different from the first dielectric material. The geometry of the intermediate dielectric element can be selected, e.g. based on electromagnetic simulations or the like, to facilitate the impedance matching function discussed above. Again, this may be considered as an impedance matching network.

An embodiment of the instrument may include a handle at the proximal end of the coaxial cable, e.g. to provide an interface to a suitable electrosurgical generator. Also, the instrument shaft may include a closed ended catheter/sheath for conveying the coaxial cable and radiating tip portion.

The localized microwave field may be substantially spherical, e.g. around the radiating tip portion or it may be elongated, e.g. a cylinder of ablation along the shaft. One advantage of a spherical field shape is that it is rotation invariant, so the orientation of the instrument in the vessel or the instrument channel does not need to be controlled.

An outer sheath may be formed over the radiating tip portion, e.g. to prevent a sharp tip damaging the wall of a blood vessel or the instrument channel of a scoping device and/or protect the instrument. The dielectric tip may have a geometry that assists manipulation of the instrument within a blood vessel. For example, the distal end of the device may be rounded, e.g. dome-like or hemispherical.

The instrument may further include a temperature sensor at the distal end thereof. The instrument can therefore provide additional feedback about the conditions at the distal end of the instrument. The temperature sensor may be a thermocouple mounted on the outer conductor of the coaxial cable or even on the radiating tip. There may be a plurality of thermocouples positioned around the radiating tip. The thermocouple(s) may be located near a tuning stub or a plurality of stubs, the stub(s) being arranged to filter out a signal having the same frequency as the microwave energy or to force the voltage at or close to the thermocouple to zero or close to zero to ensure that the response (in mV/C or V/C) of the thermocouple is not affected by the microwave signal. To avoid the microwave energy from swamping response signals from the temperature sensor, temperature measurements may also be taken when the microwave energy is off, i.e. in an OFF period of the pulsed operation. Alternatively or additionally, the instrument may include a filtering arrangement for removing noise on the response signal from the temperature sensor caused by the microwave energy, i.e. post filtering may be used to remove the microwave signal (noise) from the measurement signal - a half wavelength filter or a high frequency operational amplifier with a very high common mode rejection ratio (CMRR), e.g. 100dB, may be used to filter out the common mode signal.

The filtering arrangement may include a low pass filter and a common mode injection instrumentation amplifier arranged to remove higher frequency components from the response signal.

As an alternative to the abovementioned radiating tip portion, the distal end assembly may be a vessel sealer that can seal biological vessels using a confined microwave field that can yield a well-defined seal location with low thermal margin. Moreover, the vessel sealer may include the magnetostrictive transducer in order to provide auxiliary functionality to assist vessel dividing, fine tissue cutting, and/or dissection. With these auxiliary functions, fewer device interchanges may be needed during a procedure. The vessel sealer may be used in any type of surgical procedure, but it is expected to find particular utility for non-invasive or minimally invasive procedures. For example, the device may be configured to be introduced to a treatment site through an instrument channel of a surgical scoping device, such as a laparoscope or an endoscope.

Specifically, the distal end assembly includes a pair of jaws that are movable relative to each other to open and close a gap between opposing inner surfaces thereof, the pair of jaws comprising an energy delivery structure arranged to emit the EM energy (e.g. microwave EM energy) into the gap between the opposing inner surfaces, wherein the energy delivery structure comprises a microstrip antenna mounted on the inner surface of one or both of the pair of jaws.

The energy delivery structure may be arranged to confine an emitted microwave field substantially within a region between the pair of jaws. Accordingly, the energy delivery structure in the pair of jaws operates to provide a localised vessel seal for a biological vessel gripped between the jaws.

The distal end assembly may include a blade comprising the transducer for cutting through biological tissue, the blade being slidably disposed within the distal end assembly to be movable through the region between the pair of jaws. In this way, the blade is operable to cut through the localised vessel seal formed by the energy delivery structure and divide the vessel. The transducer may be connected to a proximal end portion of the blade.

Also, instead of forming part of a blade, the magnetostrictive transducer may be housed in or on one of the jaws. For example, the pair of jaws may comprise a first (e.g. active) jaw having the energy delivery structure mounted therein, and a second (e.g. passive) jaw which does not receive an EM energy (e.g. RF or microwave EM energy) feed, and wherein the transducer is housed in or on the second jaw. Alternatively, the transducer may be within a volume of, or on a surface of, the first jaw. For example, the transducer may be incorporated into the microstrip antenna. Specifically, the microstrip antenna may be a coplanar microstrip antenna comprising: a planar dielectric substrate having a top surface that is exposed at the gap between the opposing inner surfaces, and an under surface on an opposite side of the planar dielectric substrate from the top surface; a ground conductor layer on the under surface; a ground conductive strip on the top surface and electrically connected to the ground conductor layer; and an active conductive strip on the top surface, the active conductive strip being spaced from the ground conductive strip, wherein the active conductive strip and the ground conductive strip are positioned to have a uniform closest spacing within the region between the pair of jaws, and wherein the transducer is positioned on the top surface of the planar dielectric substrate and in-between the active conductive strip and the ground conductive strip. In an embodiment, multiple magnetostrictive transducers may be positioned in-between the active conductive strip and the ground conductive strip. In this way, the combined ultrasonic vibrations provided by multiple smaller transducers positioned in-between the active and ground conductive strips may be more comparable to a larger single transducer positioned in or on a jaw (e.g. within a volume of the jaw).

Where the transducer is housed in or on one of the jaws, a blade may not be provided. Alternatively, however, a blade may be present in addition, but the blade may provide a different type of cutting mechanism to the ultrasound transducer, for example, the blade may comprise a rigid element with a sharp edge adapted to slice biological tissue, e.g. a scalpel-type blade or the like.

In use, the vessel sealer may thus perform vessel sealing and vessel dividing. Vessel sealing is typically the application of pressure to squash the walls of a biological vessel together, followed by the application of some form of thermal energy. The thermal energy is applied by dielectric heating the gripped tissue using the microwave EM energy. The applied electro-mechanical energy disrupts/denatures the tissue cells and forms an amalgam of collagen predominant in vessel walls, which effectively bonds the vessel walls together. With time, post operatively, cellular recovery and regrowth occurs to reinforce the seal further. Vessel dividing is a process of cutting through a continuous biological vessel to separate it into two pieces. It is normally performed after a vessel is first sealed. Vessel dividing is performed by the magnetostrictive transducer which may be part of a blade in-between the jaws or may be housed in or on one of the jaws.

The energy delivery structure may comprise a microwave radiator element disposed on the inner surface of one or both of the pair of jaws. For example, the pair of jaws may comprise an active jaw having the energy delivery structure mounted therein, and a passive jaw which does not receive a microwave EM energy feed. Alternatively, each jaw in the pair of jaws may have a respective energy delivery structure mounted therein. In this scenario, the distal end assembly may include a power splitter for dividing the microwave EM energy received from the coaxial transmission line between the respective energy delivery structures. In a further example, the energy delivery structure may have components that are divided between the pair of jaws, so that the pair of jaws in combination provide a microwave radiator element.

The microwave radiator element may comprise a coplanar microstrip antenna mounted on the inner surface of one or both of the pair of jaws. In one embodiment, the coplanar microstrip antenna may be mounted on an active jaw and the opposing jaw may be a passive jaw. The inner surface of the passive jaw at the gap may comprise a resilient deformable layer of electrically insulating material, e.g. silicone rubber or the like. The layer of electrically insulating material may provide a thermal barrier to inhibit propagation of heat beyond the jaws. In some cases, the deformable layer may assist in providing a substantially constant clamping force along the length of the pair of jaws.

The coplanar microstrip antenna may comprise a planar dielectric substrate having a top surface that is exposed at the gap between the opposing inner surfaces, and an under surface on an opposite side of the planar dielectric substrate from the top surface. The dielectric substrate may be made from a suitable ceramic. It may be mounted, e.g. bonded or otherwise affixed, to the active jaw. A ground conductor layer may be provided on the under surface. This may be a layer of metallisation, e.g. of copper, silver, gold or the like. On the top surface of the dielectric substrate, there may be provided a ground conductive strip that is electrically connected to the ground conductor layer, and an active conductive strip that is spaced from the ground conductive strip. The ground conductor may be electrically connected to an outer conductor of the coaxial transmission line. The active conductive strip may be connected to an inner conductor of the coaxial transmission line. The active conductive strip and the ground conductive strip may be positioned to have a uniform closest spacing within the region between the pair of jaws. The closest spacing between the active conductive strip and the ground conductive strip is the region when the emitted microwave field will be at its strongest. Accordingly, a geometry for the active conductive strip and the ground conductive strip can be selected that confines the field within the region between the jaws.

In one example, the active conductive strip may be an elongate longitudinally extending finger electrode. The ground conductive strip comprise one or more elongate portions that flank the finger electrode whereby the closest spacing comprises a elongate longitudinally extending portion along the inner surface of the pair of jaws. The ground conductive strip may flank both sides of the finger electrode. In one example, the ground conductive strip may be a U-shaped element that flanks both sides of the finger electrode and surrounds its distal end. In this example the field may be confined primarily within a region lying inwardly of the U-shaped element. Where one or more magnetostrictive ultrasound transducers are positioned in the coplanar microstrip antenna, the ultrasound transducers may be positioned in the gap in-between the finger electrode and the U-shaped element.

The ground conductive strip may be electrically connected to the ground conductor layer via through holes formed in the dielectric substrate.

The microwave radiator element need not be limited to a coplanar microstrip configuration. In other examples it may comprise a travelling wave antenna, or meandering or interdigitated microstrip arrangement.

The opposing inner surfaces of the pair of jaws may include textured or ridged portions to retain biological tissue within the gap. This feature may also permit gas or vapour generated by the denaturing process at the sealing interface to escape.

The pair of jaws may be pivotable relative to each other about a hinge axis that lies transverse to a longitudinal axis of the coaxial transmission line. In one example, the pair of jaws comprises a static jaw that is fixed relative to the instrument shaft, and a movable jaw that is pivotably mounted relative to the static jaw to open and close the gap between the opposing inner surfaces. The energy delivery structure may be disposed on the inner surface of the static jaw. In another example, both jaws are arranged to pivot with respect to the instrument shaft, e.g. in a symmetrical forceps-type arrangement. Relative movement of the pair of jaws may be controlled from a handle at a proximal end of the instrument shaft. A control rod or control wires may pass through the instrument shaft to operably couple an actuation mechanism on the handle to the pair of jaws.

In another example, the pair of jaws may be arranged to move relative to one another in a manner that maintains the inner surfaces thereof in an aligned, e.g. parallel, orientation. This configuration may be desirable for maintaining a uniform pressure on grasped tissue along the length of the jaws. One example of such a closure mechanism is disclosed in WO 2015/097472.

When present, the blade may be slidable in a longitudinal direction between a retracted position in which it lies proximal to the pair of jaws and an extended position in which it lies within the region between the pair of jaws. It is desirable for the blade to slide into the region between the jaws when they are in a tissue gripping configuration, i.e. at least partially closed. The blade may be slidable along a longitudinally extending recessed groove formed in the pair of jaws, i.e. in each jaw of the pair of jaws, so that it can contact tissue held in the gap when the pair of jaws are closed. The groove may be arranged to act as a guide rail for the cutting blade, which may be particular useful where the pair of jaws curve towards their distal ends.

In another example, the blade may be mounted within one of the pair of jaws, and may be slidable or otherwise movable in a lateral direction between a retracted position in which it lies beneath the inner surface of the jaw and an extended position in which it lies within the region between the pair of jaws.

As mentioned above, the blade may comprise a rigid element with a sharp edge adapted to slice biological tissue, e.g. a scalpel-type blade or the like. This type of blade is configured to perform a "cold" cut, which may be preferred because it carries a low risk of collateral thermal damage that is associated with other cutting techniques. However, the invention need not be limited to a cold cut blade. In other examples, the blade may comprise the magnetostrictive transducer, a bipolar radiofrequency cutting element, and a heatable wire element. Where the magnetostrictive transducer is not part of the blade, the magnetostrictive transducer is positioned elsewhere in the vessel sealer (e.g. in one of the jaws) in order to provide an ultrasonic cutting function.

As mentioned above, the vessel sealer may advantageously provide auxiliary functions in addition to its primary microwave-based vessel sealing function, and ultrasonic-based dividing function. For example, the instrument shaft may be arranged to convey RF EM energy and the distal end assembly may be arranged to receive the RF EM energy from the instrument shaft. In this example, the distal end assembly may further comprise a dissector element arranged to deliver the RF EM energy for cutting through biological tissue, wherein the dissector element is located outside the region between the pair of jaws.

The dissector element may comprise a bipolar RF structure having an active electrode and a return electrode. The active electrode (cutting element) may be an order of magnitude smaller than the return electrode. The return electrode may be formed on an outer surface of the jaw adjacent to the dissector element, so that it is in direct contact with the tissue when used in a dry field. The dissector element may thus be used for small scale or fine cutting, e.g. to improve access to or open up a treatment site.

The cutting region may sit away from (i.e. proud) of the pair of jaws. For example, the dissector element may comprise a protruding body that presents a leading edge for contacting tissue. The active electrode may be provided at the leading edge, e.g. to ensure that the RF current density is concentrated in that region.

The dissector element may be mounted on an outer surface of the pair of jaws. For example, the protruding body may be on a distal or side surface of the pair of jaws. The protruding body may be formed from a suitable dielectric, with the active electrode being a conductive portion fabricated thereon. The return electrode may be on the protruding body or on the outer surface of the pair of jaws.

In another example, the dissector element may be mounted on a longitudinal extender, the longitudinal extender being movable longitudinally with respect to the pair of jaws. This arrangement can assist visibility of the dissector element in use, e.g. by enabling it to be moved into a treatment site before the pair of jaws.

In a preference example, the dissector element may be mounted at a distal end of the distal end assembly.

The microwave EM energy and RF EM energy may be conveyed along a common signal pathway through the instrument shaft. For example, a coaxial transmission line may provide the common signal pathway for conveying both the microwave EM energy and the RF EM energy. In this arrangement, the distal end assembly may comprise an inductive filter for blocking the microwave EM energy from the dissector element, and a capacitive filter for blocking the RF EM energy from the energy delivery structure on the pair of jaws. In an alternative arrangement, the RF EM energy and microwave EM energy are conveyed along separate pathways within the instrument shaft, wherein the inductive filter and capacitive filter are provided at a proximal end of the instrument shaft, e.g. in a handle.

As mentioned above, the distal end assembly and instrument shaft may be dimensioned to fit within an instrument channel of a surgical scoping device. The surgical scoping device may be a laparoscope or an endoscope. Surgical scoping devices are typically provided with an insertion tube that is a rigid or flexible (e.g. steerable) conduit that is introduced into a patient's body during an invasive procedure. The insertion tube may include the instrument channel and an optical channel (e.g. for transmitting light to illuminate and/or capture images of a treatment site at the distal end of the insertion tube. The instrument channel may have a diameter suitable for receiving invasive surgical tools. The diameter of the instrument channel may be equal to or less than 13 mm, preferably equal to or less than 10 mm, and more preferably, especially for flexible insertion tubes, equal to or less than 5 mm.

The vessel sealer discussed above may find applicability in other tissue welding techniques. For example, the energy delivery structure may be used as an alternative to staples. In some abdominal procedures, staple guns are used to deliver 50 to 100 small staples that are fired simultaneously between jaws that can have a length of 70 mm or more, or from an annular jawed arrangements with diameters of 20 to 50 mm. In this type of application multiple antenna structures such as those discussed herein may be used to cover the required length. The antenna structures may be arranged in any number of array forms to be activated simultaneously, sequentially or progressively in a suitable manner.

A second aspect of the invention provides an electrosurgical generator comprising: an electromagnetic (EM) signal supply unit for generating EM energy; an electrical signal supply unit for generating an electrical signal for driving a magnetostrictive ultrasound transducer (e.g. to generate ultrasonic vibrations); an output port configured to be connectable to an electrosurgical instrument for delivering the EM energy from a distal end thereof, and for generating ultrasonic vibrations using the electrical signal; and a feed structure for conveying the EM energy from the EM signal supply unit to the output port, and for conveying the electrical signal from the electrical signal supply unit to the output port, wherein the feed structure has a common signal pathway for conveying the EM energy and the electrical signal to the output port.

In this arrangement, the same generator can supply RF energy and/or microwave energy, e.g. for tissue cutting, ablation, haemostasis or other effects as well as the electrical signal for driving an ultrasound transducer (e.g. a magnetostrictive transducer) to generate ultrasonic vibrations in tissue. Ultrasonic vibrations can be used to divide, dissect, or cut biological tissue. By incorporating RF and/or microwave energy into a common generator, the invention may enable the same instrument to deliver RF and/or microwave energy as well. This may provide more treatment options for the practitioner during a treatment procedure.

As mentioned above, the electrical signal can be a varying or oscillating current signal. The varying or oscillating current signal may have one of the following forms: pulsed, sinusoidal, square, trapezoidal, ramp, exponential. In any case, the electrical signal can be applied to the coil of a magnetostrictive transducer to induce a varying magnetic field around the transducer's magnetostrictive element. Due to the magnetostrictive effect, the magnetostrictive element changes its physical dimensions with the changes in magnetic field (and the changes in current in the electrical signal). Therefore, in order to generate ultrasonic vibrations, the electrical signal includes oscillations (e.g. current oscillations) at an ultrasound frequency (e.g. 20 kHz to 5 MHz). In an embodiment, the electrical signal is an oscillating current signal which varies in amplitude by up to 100A between its minimum and maximum (e.g. between 0A and 100A).

The EM signal supply unit may be arranged to supply both RF energy and microwave energy, either separately or simultaneously. For example, the EM signal supply unit may comprise a microwave signal generator for generating microwave EM radiation having a first frequency, and a radiofrequency (RF) signal generator for generating RF electromagnetic (EM) radiation having a second frequency that is lower than the first frequency.

The feed structure may comprise an electrical signal channel for connecting the output port to the electrical signal supply unit, and a microwave channel for connecting the output port to the microwave signal generator. The electrical signal channel and microwave channel may comprise physically separate signal pathways from the electrical signal supply unit and microwave signal generator respectively. The feed structure may include a first combining circuit having: a first input connected to receive the electrical signal from the electrical signal channel, a second input connected to receive the microwave EM radiation from the microwave channel, and an output in communication with the first and second inputs for transferring the electrical signal and the microwave EM radiation to the common signal pathway.

The microwave channel may include a first filter arranged to permit the passage of microwave EM radiation from the microwave signal generator to the first combining circuit, but prevent (e.g. block) the passage of the electrical signal from the first combining circuit to the microwave signal generator. In an embodiment, the first filter may be a high-pass filter with a relatively high cut-off frequency (e.g. about 300MHz) such that it passes microwave frequency energy, but blocks the lower frequencies of the electrical signal (which has an ultrasound frequency) and any RF signal present. For example, a 1pF capacitor may be used.

Also, the electrical signal channel may comprise a second filter arranged to permit the passage of the electrical signal from the electrical signal supply unit to the first combining circuit, but prevent (e.g. block) the passage of the microwave EM radiation from the first combining circuit to the electrical signal supply unit. In an embodiment, the second filter may be a low-pass filter with a relatively high cut-off frequency (e.g. about 300MHz) such that it passes the electrical signal that has an ultrasound frequency and any RF signal present, but blocks the higher frequency microwave energy. For example, one or more (e.g. three) microwave stubs may be used, wherein the stubs are arranged to filter out a signal having the same frequency as the microwave energy.

The feed structure may comprise an RF channel for connecting the output port to the RF signal generator. The RF channel and microwave channel may comprise physically separate signal pathways from the RF signal generator and microwave signal generator respectively. Also, the RF channel may combine with the electrical signal channel. The feed structure may include a second combining circuit connected to the electrical signal channel and having: a first input connected to receive the electrical signal from the electrical signal supply unit and a second input connected to receive the RF EM radiation from the RF channel, and an output in communication with the first and second inputs for transferring the RF EM radiation and the electrical signal to the first combining circuit.

The electrical signal channel may comprise a third filter arranged to permit the passage of the electrical signal from the electrical signal supply unit to the second combining circuit, but prevent (e.g. block) the passage of the RF EM radiation from the second combining circuit to the electrical signal supply unit. In an embodiment, the third filter may be a low-pass filter which has a relatively low cut-off frequency (e.g. about 100 kHz) such that it passes the electrical signal having an ultrasound frequency, but blocks RF signals. For example, an inductor may be used.

Also, the RF channel may comprise a fourth filter arranged to permit the passage of the RF EM radiation from the RF signal generator to the second combining circuit, but prevent (e.g. block) the passage of the electrical signal from the second combining circuit to the RF signal generator. In an embodiment, the fourth filter may be a high-pass filter with a relatively low cut-off frequency (e.g. about 100 kHz) such that it passes RF frequency energy but blocks the lower ultrasound frequency of the electrical signal. For example, a $1\mu F$ capacitor may be used.

The electrical signal supply unit may include: a first power supply for outputting a first supply signal; a signal source for outputting a first control signal; a first switching circuit having a control input coupled to the signal source for receiving the first control signal, a supply input coupled to the first power supply for receiving the first supply signal, and an output, wherein the first switching circuit is operable to provide at the output at least part of the electrical signal based on the first supply signal and the first control signal. In an embodiment, the first switching circuit includes a current source which generates the electrical signal (or part thereof) from (e.g. using) the first supply signal and in accordance with a characteristic (e.g. an oscillation, a frequency, a variation) of the first control signal. For example, the current source may be a voltage controlled current source, such as, as an IGFET or MOSFET, or a current controlled current source, such as, a BJT.

The first power supply may be a DC power supply. Also, the signal source provides a varying control or trigger signal which is used to control the generation of the electrical signal from (e.g. using) the first supply signal. The signal source may be a microcontroller (e.g. an Arduino™ microcontroller), a Colpitts oscillator, a Hartley oscillator or a 555 timer. In an embodiment, the first control signal has an oscillating waveform, such as, a pulsed, sinusoidal, square, trapezoidal, ramp, or exponential waveform. In an example, the first control signal may oscillate between a LOW state (e.g. 0V) and a HIGH state (e.g. 5V) at an ultrasound frequency (e.g. 20kHz to 5MHz). The first switching circuit generates the electrical signal (or a part thereof) by generating an oscillating electrical (e.g. current) signal from the first supply signal and having the oscillations of the first control signal. For example, when the first control signal is LOW, the first switching circuit is in an OFF state such that no electrical signal is output from the electrical signal supply unit. However, when the first control signal is HIGH, the first switching circuit is in an ON state such that an electrical (e.g. current) signal is output from the electric single supply unit. This output electrical (e.g. current) signal may be generated by the current source of the first switching circuit from the first supply signal. This output electrical (e.g. current) signal may be transmitted to the electrosurgical instrument of the first aspect and used to drive its magnetostrictive transducer in order to generate ultrasonic vibrations around the distal end of the instrument for treating (e.g. dividing, cutting) biological tissue.

In the above example, the first control signal controls the first switching circuit to generate an electrical (e.g. current) signal from (e.g. using) the first supply signal. This electrical signal can be positive or negative depending on the construction of the first switching circuit and the first power supply. For example, where the first power supply is a DC power supply connected in a forward configuration and the first switching circuit comprises a P-channel MOSFET, the output electric current signal is a positive oscillating signal. On the other hand, where the first power supply is a DC power supply connected in a reverse configuration and the first switching circuit comprises an N-channel MOSFET, the output electric current signal is a negative oscillating signal. Thus, the signal source with the first power supply and the first switching circuit generate the complete electric (e.g. current) signal, which may be a positive oscillating current signal or negative oscillating current signal.

However, in another embodiment, a second power supply may be provided for outputting a second supply signal. In this case, as before, the signal source may be arranged to output the first control signal to the first switching circuit but, this time, also output a second control signal to drive a second switching circuit. The first and second control signals may be out of phase (e.g. 180 degrees) with each other such that when the first control signal is at a maximum (e.g. 5V) the second control signal is at a minimum (e.g. 0V), and when the first control signal is at a minimum (e.g. 0V) the second control signal is at a maximum (e.g. -5V). In this way, the signal source, the first power supply and the first switching circuit can provide one part (e.g. one half, or a positive half) of the electrical signal (e.g. variations between a positive maximum current and zero), and the signal source, the second power supply and the second switching circuit can provide a second (or remaining) part (e.g. the other half, or the negative half) of the electrical signal (e.g. variations between zero and a negative maximum current). Accordingly, it is possible to generate a compound electrical (e.g. current) signal with a wider amplitude range (e.g. current range) which in turn can be used to generate stronger ultrasonic vibrations. Further, since the two parts of the electrical signal are separately controlled by the first and second control signals, it is possible to introduce a delay in-between the first and second parts, which can be useful in ensuring that the coil of the magnetostrictive transducer has time to cool between cycles. In this way, wear and damage to the magnetostrictive transducer can be reduced.

It is to be understood that where the electrical signal supply unit includes the first power supply and the first switching circuit for providing the first part of the electrical signal, and the second power supply and the second switching circuit for providing the second (or remaining) part of the electrical signal, the electrical signal supply unit further includes a common signal pathway connected to the outputs of both the first and second switching circuits in order to combine the first and second parts and form the electrical signal for driving the magnetostrictive ultrasound transducer.

In an embodiment, the first and/or second power supply comprises a filtering circuit for blocking an alternating current signal or spike received at its output. Such alternating currents or spikes may be generated elsewhere in the generator and could otherwise enter the power supply at its output and cause damage to the power supply. Therefore, the filtering circuit operates to protect the power supply from such damage. In an embodiment, the filtering circuit comprises a capacitive circuit, for example, two parallel connected capacitors connected between the power supply output and zero volts. An additional advantage of such a structure is that the capacitive circuit stores power from the power supply output such that power is delivered to the rest of the circuit from the capacitive circuit rather than directly from the power supply. This capacitive circuit functions to increase the current available to the switching circuit for creating the electrical (e.g. current) signal used to drive the magnetostrictive ultrasound transducer.

In an embodiment, the first and/or second switching circuit comprises a signal conditioner coupled to a switch. The signal conditioner is operable to convert the control signal into a driving signal for operating the switch. That is, a current and/or voltage of the output from the signal source may not be large enough to charge up the inherent capacitances of the switch (e.g. a MOSFET) in order to activate the switch at ultrasound frequencies. Therefore, the signal conditioner amplifies the control signal from the signal source such that it possess a suitable size current and/or voltage for driving the switch. It is noted that the signal conditioner does not change the frequency of the control signal since the frequency is specifically set by the signal source in order to create the required ultrasonic vibrations via the magnetostrictive transducer. In an embodiment, the switch is a switchable current source, such as a voltage controlled current source (e.g. a MOSFET or IGFET) or a current controlled current source (e.g. a BJT). That is, the switch may include the aforementioned current source of the switching circuit.

It is to be understood that the first power supply may have the same physical construction as the second power supply, but the first power supply may be connected in an opposite (e.g. reverse) configuration compared to the second power supply. Also, the first switching circuit may have the same physical construction as the second switching circuit, but the first switching circuit may include a switch (e.g. MOSFET) of the opposite type compared to the second switching circuit (e.g. N-channel instead of P-channel or vice versa).

According to a third aspect of the invention, there is provided an electrosurgical apparatus comprising: an electrosurgical instrument according to the first aspect, and an electrosurgical generator according to the second aspect, wherein the output port of the electrosurgical generator is configured to be connectable to a proximal end of the instrument shaft of the electrosurgical instrument. In this way, the EM energy (e.g. microwave and/or RF) generated by the generator can be provided to the instrument in order to be delivered from the distal end assembly for tissue treatment. Also, the electrical signal generated by the generator can be provided to the instrument in order to drive the instrument's magnetostrictive ultrasound transducer to generate ultrasonic vibrations around the distal end assembly for tissue treatment.

Herein, the terms "proximal" and "distal" refer to the ends of the energy conveying structure further from and closer to the treatment site respectively. Thus, in use the proximal end is closer to a generator for providing the RF and/or microwave energy, whereas the distal end is closer to the treatment site, i.e. the patient.

The term "oscillate" is used herein to mean both regular and irregular variations.

The term "conductive" is used herein to mean electrically conductive, unless the context dictates otherwise.

The term "longitudinal" used below refers to the direction along the instrument channel parallel to the axis of the coaxial transmission line. The term "lateral" refers to a direction that is perpendicular to the longitudinal direction. The term "inner" means radially closer to the centre (e.g. axis) of the instrument channel. The term "outer" means radially further from the centre (axis) of the instrument channel.

The term "electrosurgical" is used in relation an instrument, apparatus or tool which is used during surgery and which utilises radiofrequency (RF) electromagnetic (EM) energy and/or microwave EM energy. Herein, RF EM energy may mean a stable fixed frequency in a range 10 kHz to 300 MHz, preferably in a range from 100 kHz to 5MHz, and more preferably in a range from 360 to 440 kHz. The microwave EM energy may mean electromagnetic energy having a stable fixed frequency in the range 300 MHz to 100 GHz. The RF EM energy should have a frequency high enough to prevent the energy from causing nerve stimulation. In use, the magnitude of the RF EM energy and the duration for which it is applied may be selected to prevent the energy from causing tissue blanching or unnecessary thermal margin or damage to the tissue structure. Preferred spot frequencies for the RF EM energy include any one or more of: 100 kHz, 250 kHz, 400 kHz, 500 kHz, 1 MHz, 5 MHz. Preferred spot frequencies for the microwave EM energy include 915 MHz, 2.45 GHz, 5.8 GHz, 14.5 GHz, 24 GHz. 5.8 GHz may be preferred.

## BRIEF DESCRIPTION OF DRAWINGS

Examples of the invention are described in more detail below with reference to the accompanying drawings, in which:

Fig. 1 is a schematic diagram of an electrosurgical generator in accordance with an embodiment;
Fig. 2 is a schematic diagram of an electrical signal supply unit of the electrosurgical generator of Fig. 1, in accordance with an embodiment;
Fig. 3 is a schematic diagram of an electrical signal supply unit of the electrosurgical generator of Fig. 1, in accordance with another embodiment;
Fig. 4 is a schematic diagram of an electrical signal supply unit of the electrosurgical generator of Fig. 1, in accordance with a further embodiment;
Fig. 5 is a schematic diagram of a feed structure of the electrosurgical generator of Fig. 1, in accordance with an embodiment;
Fig. 6A is a schematic diagram of a magnetostrictive ultrasound transducer in accordance with an embodiment;
Fig. 6B is a diagram of a magnetic hysteresis loop;
Fig. 7 is a schematic diagram of an electrosurgical apparatus, in accordance with an embodiment;
Fig. 8 is a schematic cross section view of an electrosurgical instrument, in accordance with an embodiment;
Fig. 9 is a schematic perspective view of an electrosurgical instrument, in an open configuration, in accordance with another embodiment;
Fig. 10 is a schematic perspective view of an underside of the electrosurgical instrument of Fig. 9;
Fig. 11 is a schematic perspective view of the electrosurgical instrument of Fig. 9, in a closed configuration;
Fig. 12A and 12B show opposing surfaces of an example coplanar microstrip antenna that can be used in the electrosurgical instrument of Fig. 9; and,
Fig. 13 shows a top surface of a coplanar microstrip antenna comprising multiple magnetostrictive ultrasound transducers, an accordance with an embodiment.

DETAILED DESCRIPTION; FURTHER OPTIONS AND PREFERENCES

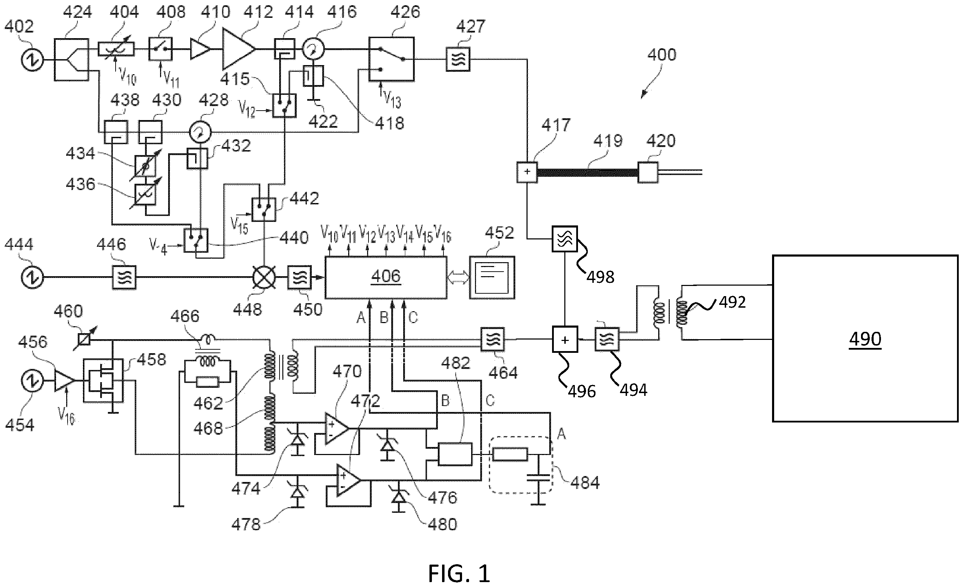

Fig. 1 shows a schematic diagram of an electrosurgical apparatus 400. The apparatus comprises a RF channel, a microwave channel, and an electrical signal channel for conveying an electrical (e.g. current) signal for driving a magnetostrictive ultrasound transducer to generate ultrasonic vibrations. The RF channel contains components for generating and controlling an RF frequency electromagnetic signal at a power level suitable for treating (e.g. cutting or desiccating) biological tissue. The microwave channel contains components for generating and controlling a microwave frequency electromagnetic signal at a power level suitable for treating (e.g. coagulating or ablating) biological tissue. The electrical signal channel contains components for generating and controlling an electrical (e.g. current) signal for driving a magnetostrictive ultrasound transducer for forming ultrasound vibrations at a power level suitable for tissue treatment (e.g. dissecting, cutting, dividing).

The microwave channel has a microwave frequency source 402 followed by a power splitter 424 (e.g. a 3 dB power splitter), which divides the signal from the source 402 into two branches. One branch from the power splitter 424 forms a microwave channel, which has a power control module comprising a variable attenuator 404 controlled by controller 406 via control signal $V_{10}$ and a signal modulator 408 controlled by controller 406 via control signal $V_{11}$, and an amplifier module comprising drive amplifier 410 and power amplifier 412 for generating forward microwave EM radiation for delivery from an instrument (e.g. probe or pair of jaws) 420 at a power level suitable for treatment. After the amplifier module, the microwave channel continues with a microwave signal coupling module (which forms part of a microwave signal detector) comprising a circulator 416 connected to deliver microwave EM energy from the source to the instrument along a path between its first and second ports, a forward coupler 414 at the first port of the circulator 416, and a reflected coupler 418 at the third port of the circulator 416. After passing through the reflected coupler, the microwave EM energy from the third port is absorbed in a power dump load 422. The microwave signal coupling module also includes a switch 415 operated by the controller 406 via control signal $V_{12}$ for connecting either the forward coupled signal or the reflected coupled signal to a heterodyne receiver for detection.

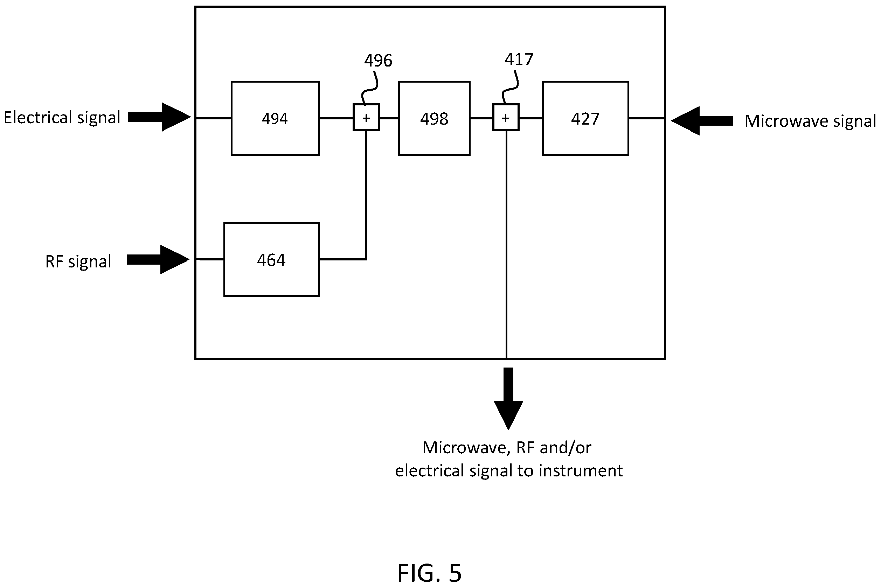

The other branch from the power splitter 424 forms a measurement channel. The measurement channel bypasses the amplifying line-up on the microwave channel, and hence is arranged to deliver a low power signal from the instrument. A primary channel selection switch 426 controlled by the controller 406 via control signal $V_{13}$ is operable to select a signal from either the microwave channel or the measurement channel to deliver to the instrument. A high band pass filter 427 is connected between the primary channel selection switch 426 and the probe 420 to protect the microwave signal generator from low frequency RF signals and/or ultrasound frequency signals (produced by electrical signal supply unit 490). The high pass filter 427 is part of a feed structure which is described in more detail below with reference to Fig. 5.

The measurement channel includes components arranged to detect the phase and magnitude of power reflected from the instrument, which may yield information about the material e.g. biological tissue present at the distal end of the instrument. The measurement channel comprises a circulator 428 connected to deliver microwave EM energy from the source 402 to the probe along a path between its first and second ports. A reflected signal returned from the instrument is directed into the third port of the circulator 428. The circulator 428 is used to provide isolation between the forward signal and the reflected signal to facilitate accurate measurement. However, as the circulator does not provide complete isolation between its first and third ports, i.e. some of the forward signal may break through to the third port and interfere with the reflected signal, a carrier cancellation circuit may be used that injects a portion of the forward signal (from forward coupler 430) back into the signal coming out of the third port (via injection coupler 432). The carrier cancellation circuit include a phase adjustor 434 to ensure that the injected portion is 180° out of phase with any signal that breaks through into the third port from the first port in order to cancel it out. The carrier cancellation circuit also include a signal attenuator 436 to ensure that the magnitude of the injected portion is the same as any breakthrough signal.

To compensate for any drift in the forward signal, a forward coupler 438 is provided on the measurement channel. The coupled output of the forward coupler 438 and the reflected signal from the third port of the circulator 428 are connected to respective input terminal of a switch 440, which is operated by the controller 406 via control signal $V_{14}$ to connect either the coupled forward signal or the reflected signal to a heterodyne receiver for detection.

The output of the switch 440 (i.e. the output from the measurement channel) and the output of the switch 415 (i.e. the output from the microwave channel) are connect to a respective input terminal of a secondary channel selection switch 442, which is operable by the controller 406 via control signal $V_{15}$ in conjunction with the primary channel selection switch to ensure that the output of the measurement channel is connected to the heterodyne receiver when the measurement channel is supplying energy to the instrument and that the output of the microwave channel is connected to the heterodyne receiver when the microwave channel is supplying energy to the instrument.

The heterodyne receiver is used to extract the phase and magnitude information from the signal output by the secondary channel selection switch 442. A single heterodyne receiver is shown in this system, but a double heterodyne receiver (containing two local oscillators and mixers) to mix the source frequency down twice before the signal enters the controller may be used if necessary. The heterodyne receiver comprises a local oscillator 444 and a mixer 448 for mixing down the signal output by the secondary channel selection switch 442. The frequency of the local oscillator signal is selected so that the output from the mixer 448 is at an intermediate frequency suitable to be received in the controller 406. Band pass filters 446, 450 are provided to protect the local oscillator 444 and the controller 406 from the high frequency microwave signals.

The controller 406 receives the output of the heterodyne receiver and determines (e.g. extracts) from it information indicative of phase and magnitude of the forward and/or reflected signals on the microwave or measurement channel. This information can be used to control the delivery of high power microwave EM radiation on the microwave channel or high power RF EM radiation on the RF channel. A user may interact with the controller 406 via a user interface 452, as discussed above.

The RF channel shown in Fig. 1 comprises an RF frequency source 454 connected to a gate driver 456 that is controlled by the controller 406 via control signal $V_{16}$. The gate driver 456 supplies an operation signal for an RF amplifier 458, which is a half-bridge arrangement. The drain voltage of the half-bridge arrangement is controllable via a variable DC supply 460. An output transformer 462 transfers the generated RF signal on to a line for delivery to the instrument 420. A high pass filter 464 is connected on that line to protect the RF signal generator from ultrasound frequency signals produced by electrical signal supply unit 490. The filter 464 also forms part of the feed structure, which is described below with reference to Fig. 5.

A current transformer 466 is connected on the RF channel to measure the current delivered to the tissue load. A potential divider 468 (which may be tapped off the output transformer) is used to measure the voltage. The output signals from the potential divider 468 and current transformer 466 (i.e. voltage outputs indicative of voltage and current) are connected directly to the controller 406 after conditioning by respective buffer amplifiers 470, 472 and voltage clamping Zener diodes 474, 476, 478, 480 (shown as signals B and C in Fig. 1).

To derive phase information, the voltage and current signals (B and C) are also connected to a phase comparator 482 (e.g. an EXOR gate) whose output voltage is integrated by RC circuit 484 to produce a voltage output (shown as A in Fig. 1) that is proportional to the phase difference between the voltage and current waveforms. This voltage output (signal A) is connected directly to the controller 406.

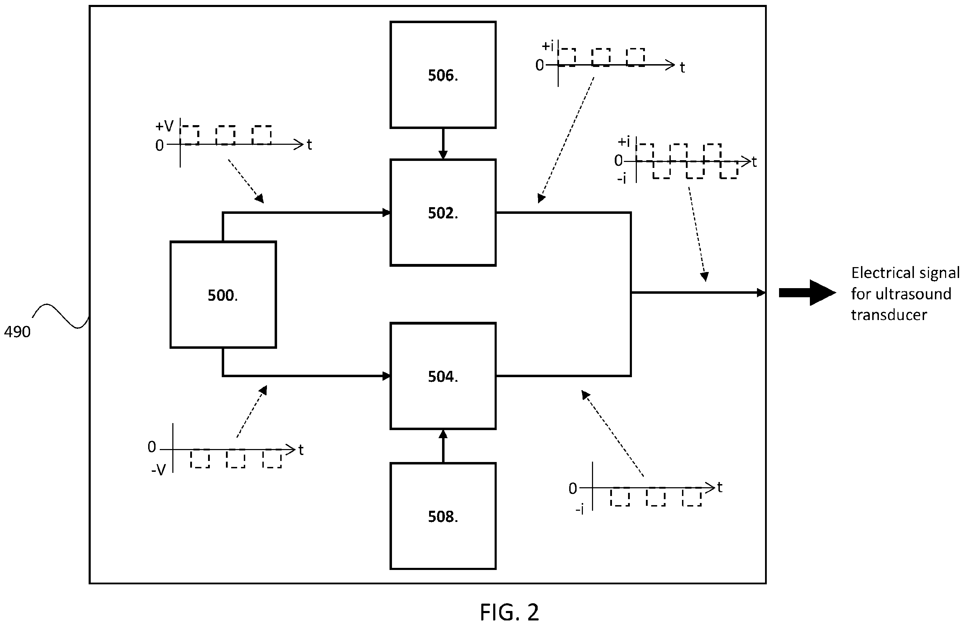

The electrical signal channel is provided in part by the electrical signal supply unit 490, an embodiment of which is shown in more detail in Fig. 2. The electrical signal supply unit 490 includes a signal source 500 for generating one or more control signals (e.g. voltage signals). In an embodiment, the signal source 500 is a microcontroller (e.g. an Arduino™ microcontroller), a Colpitts oscillator, a Hartley oscillator or a 555 timer. The signal source 500 generates control signals in the form of low power oscillating (or alternating) signals (e.g. pulsed, square, sinusoidal, ramp, trapezoidal, exponential) which drive the remaining parts of the electrical signal supply unit to form an electrical (e.g. current) signal having an ultrasound frequency for driving a magnetostrictive ultrasound transducer (e.g. on the instrument 420) so as to generate ultrasound vibrations for treating biological tissue. Specifically, the signal source 500 generates two control signals, a first (e.g. positive) control signal which is provided to a control input of a first switching circuit 502, and a second (e.g. negative) control signal which is provided to a control input of a second switching circuit 504. Example diagrammatic representations of the first and second control signals are shown in Fig. 2, wherein the first control signal is a positive square wave, and the second control signal is a negative square wave that is 180° or $\pi$ out of phase with the first control signal. The control signals are shown as voltage signals in Fig. 2 because the first and second switching circuits 502 and 504 include voltage controlled current sources, as is described in more detail below. However, it is to be understood that the control signals could be oscillating low power current signals if, for example, the first and second switching circuits 502, 504 include current controlled current sources.

In an embodiment, the first control signal oscillates at an ultrasound frequency between 0V and 5V and with minimal current (e.g. <1mA), and the second control signal oscillates at an ultrasound frequency (e.g. the same ultrasound frequency as the first control signal) between 0V and -5V and with minimal current (e.g. <1mA). The first switching circuit 502 has a supply input coupled to a first power supply 506. Also, the second switching circuit 502 has a supply input coupled to a second power supply 508. In an embodiment, the first and second power supply units may be DC power supply units. The first switching circuit 502 provides a first part of an output electrical (e.g. current) signal of the electrical signal supply unit 490. Specifically, the first switching circuit 502 comprises a current source (e.g. a MOSFET, BJT, or IGFET) and uses the first supply signal received from the first power supply 506 to generate an oscillating current signal based on the oscillations of the first control signal. This is diagrammatically shown in Fig. 2 by the positive square wave which oscillates between a current +i Amps and 0 Amps (+i Amps may be 100A in an embodiment). Also, the second switching circuit 504 provides a second or remaining part of the output electric current signal of the electrical signal supply unit 490. Specifically, the second switching circuit 504 comprises a current source (e.g. a MOSFET, BJT, or IGFET) and uses the second supply signal received from the second power supply 508 to generate an oscillating current signal based on the oscillations of the second control signal. This is diagrammatically shown in Fig. 2 by the negative square wave which oscillates between a current -i Amps and 0 Amps (-i Amps may be -100 Amps in an embodiment).

As seen on Fig. 2, the output (i.e. the first part of the electrical signal) from the first switching circuit 502 and the output (i.e. the second part of the electrical signal) from the second switching circuit 504 are combined together on a common signal path to form a combined output electrical (e.g. current) signal from the electrical signal supply unit 490. Therefore, the output from the electrical signal supply unit 490 is an electrical signal which varies at the same ultrasound frequency as the signal source 500 and between the maximum current values provided by the first and second switching circuits (i.e. between +i and -i). That is, the electrical (e.g. current) signal output from the electrical signal supply unit 490 is a compound signal made up from two portions: a first (e.g. positive) portion provided by the signal source 500, the first power supply 506 and the first switching circuit 502; and, a second (e.g. negative) portion provided by the signal source 500, the second power supply 508 and the second switching circuit 504. It is to be understood that the positive portion will cause a positive H-field in the magnetostrictive ultrasound transducer and so cause a change in dimension in one direction, whereas the negative portion will cause a negative H-field in the transducer and so cause a change in dimension in a second (e.g. opposite) direction.

An advantage of having two power supplies 506, 508 and two switching circuits 502, 504 which provide the first and second parts of the output electrical (e.g. current) signal is that the waveform of the output electrical signal can be adapted to improve performance of the ultrasound transducer. For example, the first and second control signals can be adapted to introduce a delay between the two parts of the output electrical signal in order to provide time for the transducer (e.g. its coil and/or its magnetostrictive element) to cool after being driven in one direction and before being driven in the opposite direction. In turn, this may increase the useable lifespan of the transducer and/or reduce the chances of it breaking or malfunctioning.

In an embodiment, the operation of the signal source may be controlled by the controller 406, for example, via a dedicated control signal received therefrom.

Returning to Fig. 1, a transformer 492 is coupled at one side to the electrical signal supply unit 490 and at the other side to a low pass filter 494. The transformer 492 functions to isolate the instrument 420 (and a patient) from the electric supply unit 490. In an embodiment, the transformer 492 may be an opto-isolator. Also, the transformer 492 may be absent in some embodiments. In any case, the low pass filter 464 is connected to the RF channel by signal combiner 496, and the low pass filter 494 operates to protect the electrical signal supply unit from RF signals generated on the RF channel. The filter 494 and the signal combiner 496 also form part of the feed structure, which is described below with reference to Fig. 5.

The microwave/measurement channel is connected to a signal combiner 417. Also, the signal combiner 417 is connected to the signal combiner 496 via a low pass filter 498, which functions to protect both the RF channel and the electrical signal channel from microwave energy. Further, the microwave, RF and electrical signal are conveyed separately or simultaneously along cable assembly 419 to the instrument 420. The instrument 420 delivers (e.g. radiates) microwave and/or RF energy into the biological tissue of a patient. Also, the instrument 420 includes a magnetostrictive ultrasound transducer, and the electrical signal drives the transducer to generate ultrasonic vibrations for tissue treatment.

It is to be understood that in some embodiments, only one of the microwave and RF channels may be present and so the instrument may deliver only one of RF and microwave energy. Also, the measurement channel may be absent in some embodiments.

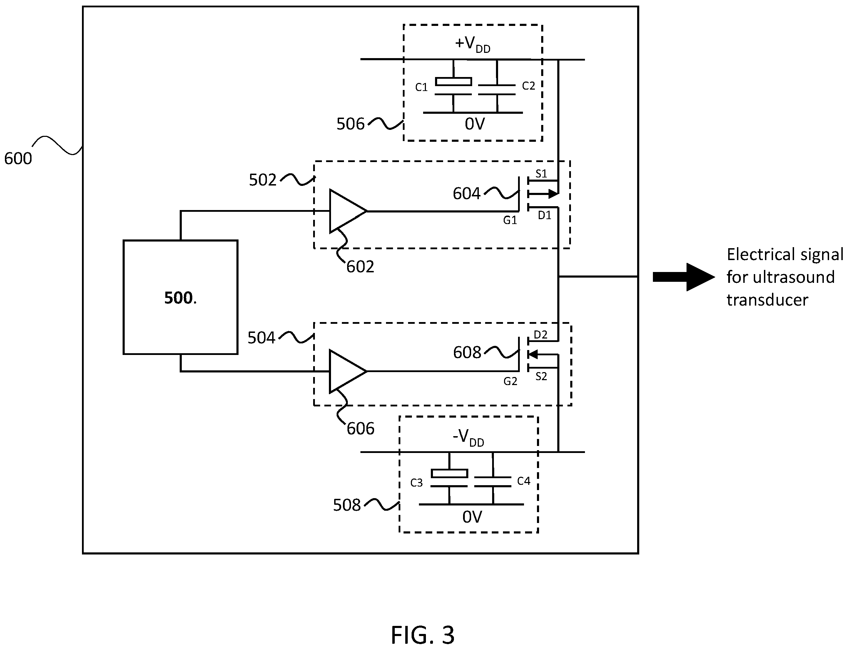

Fig. 3 shows a more detailed embodiment of the electrical signal supply unit 600. The electrical signal supply unit 600 is analogous to the electrical signal supply unit 490 of Fig. 2, wherein like reference numerals relate to like components. As is clear from Fig. 3, the electrical signal supply unit 600 includes the signal source 500. However, in Fig. 3, the first switching circuit 502 of Fig. 2 is constructed from a first gate driver 602 and a first switch 604 comprising a current source. Additionally, the second switching circuit 504 of Fig. 2 is constructed from a second gate driver 606 and a second switch 608 comprising a current source. In an embodiment, the first and second switches 604, 608 are MOSFETs (i.e. voltage controlled current sources), for example, switch 604 may be a P-channel MOSFET and switch 608 may be an N-channel MOSFET. Also, the gate drivers 602, 606 function to condition the control signals received from the signal source 500 into suitable signals for driving the switches 604, 608, respectively. For example, as mentioned previously, the first control signal is a low power oscillating positive signal which oscillates at an ultrasound frequency between, for example, 0V and 5V and with minimal current (e.g. <1mA). Also, the second control signal is a low power oscillating negative signal which oscillates at an ultrasound frequency (e.g. the same ultrasound frequency as the first control signal) between, for example, 0V and -5V and with minimal current (e.g. <1mA). However, switches 604 and 608 (e.g. MOSFETs) may require a higher voltage and current in order to operate and, therefore, the gate drivers 602 and 606 condition the control signals by increasing their voltage and/or current such that the conditioned signals can drive the switches 604, 608. For example, larger signals may be required in order to charge-up inherent capacitances of the MOSFETs. In an embodiment, the gate driver 602 may condition the first control signal into an oscillating signal which oscillates between 0V and 15V and with a current of 1A. Also, the gate driver 606 may condition the second control signal into an oscillating signal which oscillates between 0V and -15V and with a current of 1A. The gate drivers 602, 606 may be referred to as signal conditioners.

Also, in Fig. 3, the first power supply 506 of Fig. 2 includes a capacitive circuit C1, C2 which performs two functions. Also, the second power supply 508 of Fig. 2 includes a capacitive circuit C3, C4 which performs the same two functions. Firstly, the capacitive circuits (C1, C2 and C3, C4) provide power supply decoupling that prevents alternating current (AC) or spikes from entering the output of the DC power supplies, i.e. it takes such AC currents or spikes to ground. This reduces the chances that the DC power supply will be damaged by such AC currents or spikes. Secondly, the capacitive circuits (C1, C2 and C3, C4) cause the output electrical signal from the electrical signal supply unit 600 to be provided from the capacitive circuits rather than directly from the power supplies. In turn, this increases the current available for generating the output electrical (e.g. current) signal. In an embodiment, capacitors C1 and C3 may each have a value of 100μF, whereas capacitors C2 and C4 may each have a value of 0.1 μF.

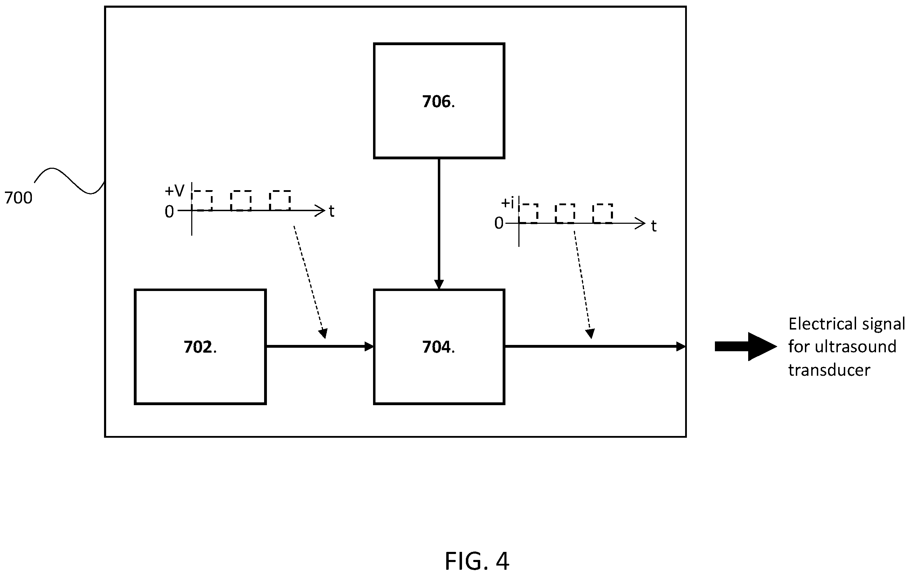

Fig. 4 shows an alternative embodiment of the electrical signal supply unit 700. Specifically, comparing the electrical signal supply unit 700 with the electrical signal supply unit 490 of Fig. 2, it is clear that the unit 700 includes a signal source 702 which generates a single control signal for a single switching circuit 704. Also the switching circuit 704 receives a supply signal from a single power supply 706. As such, the output electrical (e.g. current) signal provided by the electrical signal supply unit is either positive (e.g. between 0 and +i) or negative (e.g. between 0 and -i). This is in contrast to the arrangement of Fig. 2 in which the output electrical (e.g. current) signal is a compound signal constructed from a two parts (e.g. a positive part and a negative part). Whilst the arrangement of Fig. 4 is simpler and cheaper to manufacture, it can provide a smaller magnitude range compared to the arrangement of Fig. 2, since it tends to generate either a positive or a negative signal. Additionally, since the output electrical signal is not a combination of two control signals, it is not possible to use the control signals to introduce a delay between the positive and negative portions of the output electrical signal.

A further embodiment of the electrical signal supply unit maybe formed as a variant of the electrical signal supply unit of Fig. 3, wherein a single gate driver may receive a single control signal from the signal source 500, and the single gate driver may condition that single control signal to drive both the switch 604 and the switch 608. In this way, advantageously, the output electrical signal may have the wider current range of the arrangement of Fig. 2 (e.g. +i to -i). However, as with the arrangement of Fig. 4, this variant would be unable to introduce a delay between the positive and negative portions of the output electrical signal. That is, the conditioned control signal from the single gate driver must switch the switch 604 ON at the same time as switching the switch 608 OFF (and vice versa).

Fig. 5 shows a schematic view of a feed structure of the generator in accordance with the embodiment of Fig. 1. The feed structure receives as inputs a microwave EM signal from the microwave channel, an electrical signal for driving the ultrasound transducer from the electrical signal channel, and an RF EM signal from the RF channel. The feed structure provides as an output one or more of the three input signals (separately or simultaneously) for onward transmission to the instrument 420 via the feed line 419. The feed structure includes one or more signal combiners and one or more filters in order to combine together these different signals in such a way as to avoid damage to the separate but interconnected mechanisms for generating these different signals. Specifically, the feed structure includes a first signal combiner 417 which combines the microwave channel and the electrical signal channel into a common signal path. Additionally, the feed structure includes a second signal combiner 496 for combining the RF channel with the electrical signal channel. The RF EM signal and the electrical signal may cause damage to the mechanisms for generating the microwave EM signal and, therefore, positioned in the microwave channel and before the signal combiner 417 is the high-pass filter 427 with a relatively high cut-off frequency (e.g. about 300 MHz). This high pass filer 427 passes microwave EM frequency energy, but blocks the lower frequencies of the electrical signal (which has an ultrasound frequency) and the RF signal. For example, a 1pF capacitor may be used as the filter 427.

Additionally, the microwave EM signal may cause damage to the mechanisms for generating the electrical signal and the RF EM signal and, therefore, positioned in-between the first and second signal combiners 417, 496 is the low-pass filter 498 with a relatively high cut-off frequency (e.g. about 300 MHz) such that it passes the electrical signal that has an ultrasound frequency and the RF EM signal, but blocks the higher frequency microwave energy. For example, one or more (e.g. three) microwave stubs may be used, wherein the stubs are arranged to filter out a signal having the same frequency as the microwave energy. The stubs may be as disclosed in WO2017103209A1.

Additionally, the RF EM signal may cause damage to the mechanisms for generating the electrical signal and, therefore, positioned in the electrical signal channel and before the signal combiner 496 is the low-pass filter 494 which has a relatively low cut-off frequency (e.g. about 100 KHz) such that it passes the electrical signal having an ultrasound frequency, but blocks RF signals. For example, an inductor may be used. Also, the electrical signal may cause damage to the mechanisms for generating the RF EM signal and, therefore, positioned in the RF channel and before the signal combiner 496 is the high-pass filter 464 with a relatively low cut-off frequency (e.g. about 100 kHz) such that it passes RF frequency energy but blocks the lower ultrasound frequency of the electrical signal. For example, a 1μF capacitor may be used as the filter 464.

As such, the feed structure conveys EM energy (e.g. microwave and/or RF) from respective EM energy supply units and conveys the electrical signal from the electrical signal supply unit. Also, the feed structure includes a common signal pathway for conveying the EM energy and the electrical signal to the output port for onward transmission to the instrument 420. Moreover, the feed structure includes a circuit or network of signal combiners and filters which function to ensure that these separate and different input signals do not damage the various separate and different mechanisms for producing those signals.

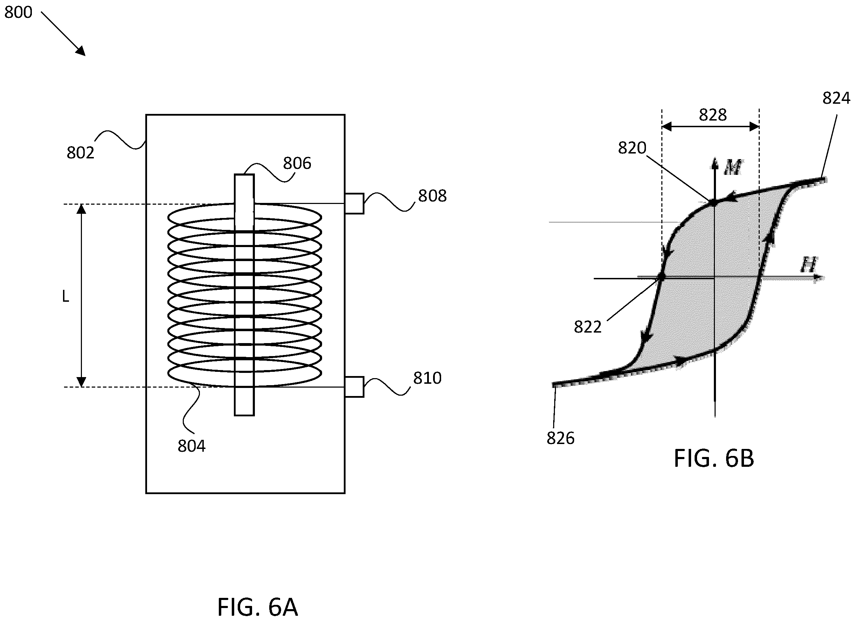

Fig. 6A illustrates a magnetostrictive ultrasound transducer in accordance with an embodiment. The transducer includes a housing 802 within which is located a coiled conductor 804 which is wrapped around an element 806 of magnetostrictive material. The element 806 may be substantially elongate or rod shaped, and may be referred to as a solenoid. In an embodiment, the element 806 may be approximately the same length as the coil, or just longer than the coil, for example, 1cm or 1.2cm. Also, the element 806 may be substantially cylindrical and have a diameter of about 0.5cm or 0.6cm. The coiled conductor 804 is connected at a first end to a first terminal 808 of the transducer 800, and at a second end to a second terminal 810 of the transducer 800. In use, the first terminal 808 and the second terminal 810 are connected to the cable assembly 419 of Fig. 1 in order that the electrical signal is provided to the transducer 800 so that the transducer 800 generates ultrasonic vibrations. Specifically, magnetostriction is a property of ferromagnetic materials which causes them to expand or contract (i.e. change their physical dimensions) in response to a magnetic field (H-field). This effect allows magnetostrictive materials to convert electromagnetic energy into mechanical energy. As a magnetic field is applied to the material, its molecular dipoles and magnetic field boundaries rotate to align with the field. This causes the material to strain and elongate.

Fig. 6B shows an example magnetic hysteresis loop. A magnetic hysteresis loop is produced when a ferromagnetic material is magnetised in one direction, until it reaches a saturation point (e.g. point 824 on Fig. 6B), then the magnetic field strength is demagnetised in the opposite direction to the reverse saturation point (e.g. point 826 on Fig. 6B). When the magnetic field direction is alternated, a loop is formed which goes back and forth from the saturation point and reverse saturation point. This loop can be viewed in Fig. 6B. The magnetising field (H) provides the required stress to produce the required change in magnetisation (M or B) or the strain. The magnetisation (M) of the ferromagnetic material when the magnetic field strength (H) is at zero is called remanence. As the magnetisation falls back to zero, the magnetic field strength (H) needed to demagnetise after saturation is called the coercivity. The point marked 820 can be referred to as the remanence point and is a measure of the remaining magnetisation when the driving field is dropped to zero. This illustrates that when the driving magnetic field drops to zero, the ferromagnetic material retains a considerable degree of magnetisation. The point marked 822 can be referred to as the coercivity point and is a measure of the reverse field needed to drive the magnetisation to zero after being saturated. This illustrates that the driving magnetic field must be reversed and then increased to drive the magnetisation to zero again. Point 824 indicates a point at which the ferromagnetic material is magnetised to saturation by alignment of magnetic domains in one direction (e.g. north pole), whereas the point 826 indicates a point at which the ferromagnetic material is magnetised to saturation by alignment of magnetic domains in the opposite direction (e.g. south pole). A width 828 of the hysteresis loop indicates if a material retains a large or small fraction of the saturation field when the driving field is removed. The width may vary between different magnetostrictive materials. A narrow hysteresis loop implies that a small amount of dissipated energy is repeatedly reversing the magnetisation. Embodiments are directed to the use of magnetostrictive materials in the production of ultrasound vibrations. Given the relatively high frequency of ultrasound vibrations, the switching between saturation points must occur relatively quickly, e.g. between 20,000 and 5,000,000 times per second (i.e. 20 kHz to 5 MHz). Therefore, an aim is to keep the hysteresis loop as narrow as possible (i.e. to keep width 828 as low as possible) so that the change (or switch) of the magnetisation occurs at a much faster rate and so that energy dissipation and subsequent heating remains small.

Terfenol-D has been selected as a particularly suitable magnetostrictive material from which to make the element 806. Terfenol-D is a good material to use because it possesses a large magnetisation and magnetostriction at room temperature, which is about 2400ppm, which occurs due to rhombohedral distortion of the lattice structure of the Terfenol-D material. That is, Terfenol-D, relative to other magnetostrictive materials, produces a large strain (i.e. change in physical dimension) for a given stress (i.e. applies change in magnetic driving field). For example, Galfenol has a magnetostriction of about 400ppm and Alfenol has a magnetostriction of about 200ppm. However, since Terfenol-D is a relatively expensive material, the element 806 may be constructed from two portions: a first portion that is substantially the same length as the coil and is made from Terfenol-D, and a second portion that is an extension of the first portion and is made from a cheaper material, such as, steel.

The following provides some example calculations to determine a possible structure of the coil 804 and element 806, in accordance with an embodiment.

It is known that the magnetic field strength (H) can be found from equation (1) below:

$$H = \frac{NI}{L} \quad (1)$$

where $N$ refers to the number of turns of wire on the coil 804, $I$ is the current of the electrical signal used to drive the transducer 800, and $L$ is the length of the coil 804 (labelled "L" in Fig. 6A).

In an embodiment, it may be considered that a magnetic field strength of 2000 Oersted (Oe) will produce a maximum strain of 2400ppm for a certain Terfenol-D magnetostrictive element 806. If 1Oe is equivalent to 79.58A/m, then 2000 Oe is equivalent to 159,155A/m.

From equation (1), if a current of 100A is driven into the coil 804, then N/L = 1,592. N/L is the ratio of the number of turns of wire on the coil 804 divided by the length (L) of the coil 804. If the length of the coil is 1cm (0.01 m), then the number of turns required will be: (N = H*L)/I = (159155*0.01)/100 = 15.9 turns - i.e. about 16 turns.

In an embodiment, the coil is made from copper wire with an outer diameter of 0.5mm, and so it is possible to get 20 turns within a 1cm length and a single layer winding. Additionally, it is possible to have multi-layer windings. However, it should be noted that the number of turns in an opposite direction should be minimised in order to avoid having a cancelling-out effect. For example, a double layer winding for providing 20 turns could include 11 turns in a first direction (layer 1), a single return turn (which would cancel out one of the original turns - i.e. the 11 turns reduces to 10 turns) (layer 2), followed by 10 more turns in the first direction (layer 3). Using multi-layer windings in this manner makes it possible to reduce the coil length required and, therefore, reduce the overall size of the transducer 800. For example, the coil length could be reduced to 0.5cm or less. Another mechanism for reducing the coil length required and, therefore, reduce the overall size of the transducer 800, is to increase the current of the driving electrical signal applied to the input terminals, 808, 810 of the transducer 800. For example, considering the arrangement of Fig. 3, this could be done by increasing the size of the power supplies (e.g. +$V_{DD}$,-$V_{DD}$) and the size of the current sources (e.g. MOSFETs).

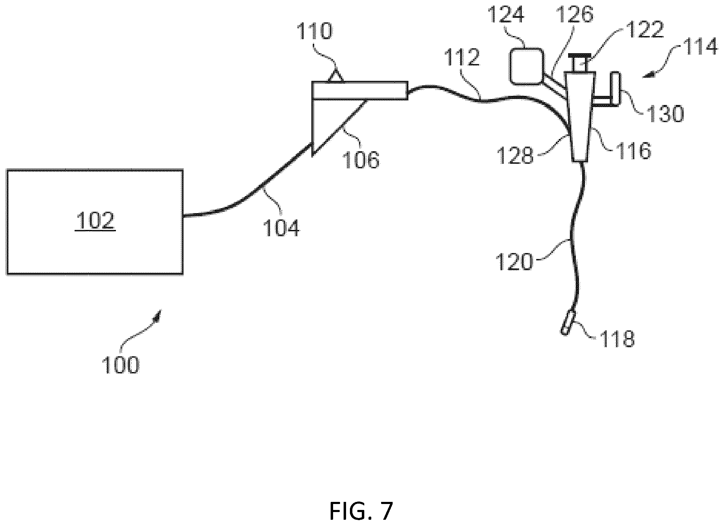

Fig. 7 is a schematic diagram of a complete electrosurgery system (or apparatus) 100 that is capable of supplying RF energy, microwave energy, or an electrical signal for driving a magnetostrictive ultrasound transducer to generate ultrasound vibrations. The system 100 comprises a generator 102 for controllably supplying RF energy, microwave energy, and the electrical signal suitable for driving a magnetostrictive ultrasound transducer to generate ultrasound vibrations. In an embodiment, the generator 102 is the same as generator 400 described above with reference to Figs. 1 to 5.

The generator 102 is connected to an interface joint 106 by an interface cable 104. If needed, the interface joint 106 can house an instrument control mechanism that is operable by sliding a trigger 110, e.g. to control longitudinal (back and forth) movement of one or more control wires or push rods (not shown). If there is a plurality of control wires, there may be multiple sliding triggers on the interface joint to provide full control. The function of the interface joint 106 is to combine the inputs from the generator 102 and instrument control mechanism into a single flexible shaft 112, which extends from the distal end of the interface joint 106.

The flexible shaft 112 is insertable through the entire length of an instrument (working) channel of a surgical scoping device 114, such as an endoscope, laparoscope, bronchoscope, gastroscope or the like.

The surgical scoping device 114 comprises a body 116 having a number of input ports and an output port from which an instrument cord 120 extends. The instrument cord 120 comprises an outer jacket which surrounds a plurality of lumens. The plurality of lumens convey various things from the body 116 to a distal end of the instrument cord 120. One of the plurality of lumens is an instrument channel. Other lumens may include a channel for conveying optical radiation, e.g. to provide illumination at the distal end or to gather images from the distal end. The body 116 may include an eye piece 122 for viewing the distal end. In order to provide illumination at the distal end, a light source 124 (e.g. LED or the like) may be connected to the body 116 by an illumination input port 126.

The flexible shaft 112 has a distal assembly 118 (not drawn to scale in Fig. 7) that is shaped to pass through the instrument channel of the surgical scoping device 114 and protrude (e.g. inside the patient) at the distal end thereof. The distal end assembly includes an active tip for delivering microwave energy into biological tissue as discussed herein. The distal assembly 118 may be analogous to the instrument 420 of Fig. 1, and the flexible shaft 112 may be analogous to the feed line 419 of Fig. 1.

The structure of the distal assembly 118 discussed below may be designed to have a maximum outer diameter equal to or less than 2.0 mm, e.g. less than 1.9 mm (and more preferably less than 1.5 mm) and the length of the flexible shaft can be equal to or greater than 1.2 m.

The body 116 includes a power input port 128 for connecting to the flexible shaft, which comprises a coaxial cable (e.g. a conventional coaxial cable) capable of conveying the microwave energy, RF energy and an electrical signal from the generator 102 to the distal assembly 118. Alternatively, different means may be provided to conveying one or more of these signals. For instance, the microwave energy may be conveyed by the coaxial cable, but the electrical signal and/or the RF energy may be conveyed by a twisted cable pair or the like. Coaxial cables that are physically capable of fitting down the instrument channel of a surgical scoping device are available with the following outer diameters: 1.19 mm (0.047"), 1.35 mm (0.053"), 1.40 mm (0.055"), 1.60 mm (0.063"), 1.78 mm (0.070"). Custom-sized coaxial cables (i.e. made to order) may also be used.

As discussed above, it is desirable to be able to control the position of at least the distal end of the instrument cord 120. The body 116 may include a control actuator 130 that is mechanically coupled to the distal end of the instrument cord 120 by one or more control wires (not shown), which extend through the instrument cord 120. The control wires may travel within the instrument channel or within their own dedicated channels. The control actuator 130 may be a lever or rotatable knob, or any other known catheter manipulation device. The manipulation of the instrument cord 120 may be software-assisted, e.g. using a virtual three-dimensional map assembled from computer tomography (CT) images.

The coaxial cable for delivering the microwave radiation to the target site should be low-loss, have a small cross-section and be flexible. The cable should be low loss to avoid or reduce heating during treatment and so that there is enough power at the distal end to produce the desired radiation from the antenna.

If the cable is not separated from the body by the use of a sealed scoping device, catheter or other protective sheath, then the cable should be made of, or be coated with, a biologically inert material to avoid unwanted interaction with the body.

A preferred cable type is a coaxial cable which is made up of an inner conductor axially surrounded by a dielectric sheath which is in turn axially surrounded by an outer conductor. The radiating portion in an antenna produced from such a cable may be made up of a section of inner conductor and dielectric sheath which protrudes from the end of the outer conductor of the coaxial cable.

In an embodiment, the outer conductor of the coaxial cable may be as physically thick as possible to increase its thermal mass and heat capacity. In this way, all or a majority of the heat generated in the cable due to conveying microwave energy can be held within the structure of the cable rather than, for example, being leaked inside the patient. In an embodiment, the outer conductor may be 0.5 mm thick.

The invention also seeks to provide an antenna with a well-defined radiation pattern. It is desirable that a practitioner would be able to select an instrument for the treatment of a specific area of tissue, such that the radiation of target tissue is maximised and the radiation of healthy tissue is minimised. For example, in some circumstances it can be desirable to produce a generally spherically symmetric radiation pattern with a substantially uniform power absorption distribution, so that the amount of radiation received by an area of tissue can be more easily controlled by the practitioner.

It is also preferable that the instrument can be operated alongside other instruments to enable practitioners to receive information from the target site. For example, a scoping device may aid the steering of the instruments around obstacles within a patient's body. Other instruments may include a thermometer or camera.

In the following description, unless stated otherwise, the length of a component refers to its dimension in the direction parallel to the longitudinal axis of the coaxial cable.

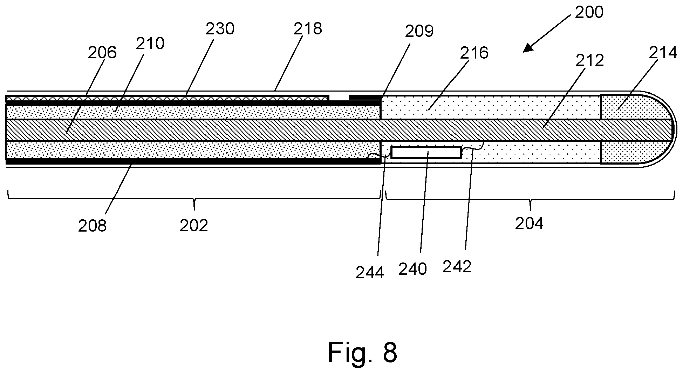

Fig. 8 is a cross-sectional view of the distal end of an electrosurgical instrument 200 that is an embodiment of the invention. The electrosurgical instrument 200 may include the distal assembly 118 of Fig. 7, or the instrument 420 of Fig. 1. The electrosurgical instrument 200 may therefore be used to deliver microwave energy and/or RF energy into biological tissue for tissue treatment. Also, the instrument 200 includes a magnetostrictive ultrasound transducer for converting an electrical (e.g. current) signal into ultrasonic vibrations in biological tissue for tissue treatment. The electrosurgical instrument 200 comprises a coaxial cable 202 that is connected at its proximal end to an electrosurgical generator (e.g. generator 400 of Fig. 1 or generator 102 of Fig. 7) in order to convey microwave energy. The coaxial cable 202 comprises an inner conductor 206, which is separated from an outer conductor 208 by a first dielectric material 210. The coaxial cable 202 is preferably low loss for microwave energy. A choke (not shown) may be provided on the coaxial cable to inhibit back propagation of microwave energy reflected from the distal end and therefore limit backward heating along the device.

The device may include a temperature sensor at the distal end. For example, in Fig. 8 a thermocouple 230 is mounted on the outer conductor to transmit a signal back to the proximal end that is indicative of temperature at the distal end of the instrument.

Other techniques for temperature monitoring can be used. For example, one or more micromechanical structures whose physical configuration is sensitive to temperature may be mounted in the distal portion of the device, e.g. in or on the outer sheath discussed below. These structures can be interfaced with an optical fibre, whereby changes in a reflected signal caused by movement of the structure can be indicative of temperature changes.

The coaxial cable 202 terminates at its distal end with a radiating tip section 204. In this embodiment, the radiating tip section 204 comprises a distal conductive section 212 of the inner conductor 206 that extends beyond a distal end 209 of the outer conductor 208. The distal conductive section 212 is surrounded at its distal end by a dielectric tip 214 formed from a second dielectric material, which is different from the first dielectric material 210. The length of the dielectric tip 214 is shorter than the length of the distal conductive section 212. An intermediate dielectric sleeve 216 surrounds the distal conductive section 212 between the distal end of the coaxial cable 202 and the proximal end of the dielectric tip 214. The intermediate dielectric sleeve 216 is formed from a third dielectric material, which is different from the second dielectric material but which may be the same as the first dielectric material 210.

In this embodiment, the coaxial cable 202 and radiating tip section 204 have an outer sheath 218 formed over their outermost surfaces. The outer sheath 218 may be formed from a biocompatible material. The outer sheath 218 has a thickness that is small enough to ensure that it does not significantly interfere with the microwave energy radiated by the radiating tip section 204 (i.e. radiating pattern and return loss). In an embodiment, the sheath is made from PTFE, although other materials are also appropriate. The thickness of the wall of the sheath is selected to withstand breakdown voltages equal to or greater than 200 kV/m.

The purpose of the dielectric tip 214 is to alter the shape of the radiated energy. The second dielectric material is selected to reduce the wavelength of the microwave energy, which results in the radiated energy exhibiting a more spherical radiation pattern. To do this, the second dielectric material preferably has a large dielectric constant (relative permittivity $\varepsilon_r$). The dielectric constant of the second dielectric material is preferably chosen to enable the length of the dielectric tip 214 to be minimised whilst still constituting a non-negligible portion of a wavelength of the microwave energy when it propagates through the second dielectric material. It is desirable for the dielectric tip 214 to be as short as possible in order to retain flexibility in the device, especially if the second dielectric material is rigid. In an embodiment, the dielectric tip 214 may have a length equal to or less than 2 mm. The dielectric constant of the second dielectric material may be greater than 80, and is preferably 100 or more at the frequency of the microwave energy. The second dielectric material may be TiOz (titanium dioxide).

The wavelength of radiation in a material becomes shorter as the dielectric constant of the material increases. Therefore a dielectric tip 214 with a greater dielectric constant will have a greater effect on the radiation pattern. The larger the dielectric constant, the smaller the dielectric tip 214 can be while still having a substantial effect on the shape of the radiation pattern. Using a dielectric tip 214 with a large dielectric constant means that the antenna can be made small and so the instrument can remain flexible. For example the dielectric constant in TiOz is around 100. The wavelength of microwave radiation having a frequency of 5.8 GHz is about 6 mm in TiOz compared to around 36 mm in PTFE (which may be the material used for the first and/or third dielectric materials). A noticeable effect on the shape of the radiation pattern can be produced in this arrangement with a dielectric tip 214 of approximately 1 mm. As the dielectric tip 214 is short, it can be made from a rigid material whilst still maintaining flexibility of the antenna as a whole.

The dielectric tip 214 may have any suitable distal shape. In Fig. 8 it has a dome shape, but this is not necessarily essential. For example, it may be cylindrical, conical, etc. However, a smooth dome shape may be preferred because it increases the mobility of the antenna as it is manoeuvred through small channels (e.g. inside blood vessels). The dielectric tip 214 may be coated with a non-stick material such as Parylene C or Parylene D, or PFTE to prevent the tissue from sticking to the instrument. The whole instrument can be coated in this way.

The properties of the intermediate dielectric sleeve 216 are preferably chosen (e.g. through simulation or the like) so that the radiating tip section 204 forms a quarter wave impedance transformer for matching the input impedance of the generator into a biological tissue load in contact with the radiating tip section 204.

During treatment, the surrounding tissue absorbs the radiated energy. The volume of tissue into which the energy is delivered depends on the frequency of the microwave energy.

As seen on Fig. 8, the radiating tip portion 204 includes magnetostrictive transducer 240 which is coupled to the inner conductor 206 (e.g. directly or via distal conductive section 212) by a first connector 242 and which is coupled to the outer conductor 208 by a second connector 244. In the embodiment of Fig. 8, the transducer 240 is encased (e.g. partly or completely) in the intermediate dielectric sleeve 216; however, it is to be understood that the transducer 240 may instead be positioned on an inner or outer surface of the intermediate dielectric sleeve 216. Further, the transducer 240 may alternatively be positioned elsewhere on the radiating tip portion, for example, in or on the radiating tip 214. In an embodiment, the transducer 240 has the same or similar construction to the transducer 800 of Fig. 6A.

In use, an electrical (e.g. current) signal for driving the transducer 240 may be introduced into the coaxial cable 202 at its proximal end (e.g. by generator 102, or generator 400). As described above with reference to Figs. 1 to 5, the electrical signal may oscillate at an ultrasound frequency. On receiving this electrical signal, the coil of the transducer 240 induces an oscillating magnetic field around the magnetostrictive element the transducer 240 such that the magnetostrictive effect causes the magnetostrictive element to rapidly expand and contract at the ultrasonic frequency thereby generating ultrasonic vibrations. Since the transducer 240 is coupled to the radiating tip portion 204, these ultrasonic vibrations travel into the radiating tip portion and are then radiated out of the instrument 200 and into the surrounding biological tissue. The vibrations produce mechanical friction that generates thermal energy thereby resulting in extracellular heating followed subsequently by intracellular heating. In this way, the ultrasound vibrations can be used to treat (e.g. cut or coagulate) the biological tissue.

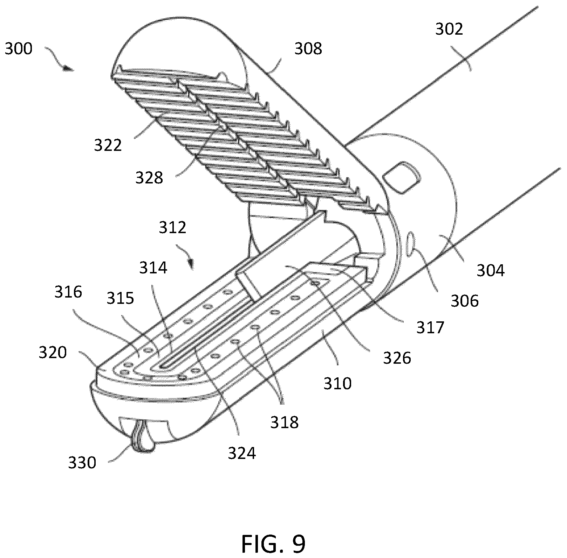

A further embodiment of an electrosurgical instrument will now be described with reference to Figs. 9 to 12B, wherein the instrument comprises an electrosurgical vessel sealer device capable of delivering microwave energy, RF energy and ultrasonic vibrations to seal blood vessels. The electrosurgical vessel sealer may include the distal assembly 118 of Fig. 7, or the instrument 420 of Fig. 1. The electrosurgical vessel sealer may be used in open surgery, but may find particular use in procedures where there is restricted access to the treatment site. For example, the electrosurgical vessel sealer may be adapted to fit within the instrument channel of a surgical scoping device i.e. laparoscope, endoscope, or the like, as described above with reference to the scoping device of Fig. 7.

Fig. 9 shows a schematic perspective view of a distal end assembly 300 of an electrosurgical instrument that is an embodiment of the invention. The distal end assembly 300 is connected to an instrument shaft 302 which is dimensioned to fit within the instrument channel of a laparoscope or other surgical scoping device. The instrument shaft 302 comprises a tubular sheath that conveys a coaxial cable for carrying EM power (e.g. microwave and/or RF) to the distal end assembly together with various control wires or rods that are arranged to control physical manipulation of the distal end assembly, as discussed below.

In this example, the distal end assembly 300 comprises a pair of jaws 308, 310. The jaws 308, 310 are operably coupled to a collar 304 that is mounted on a distal end of the instrument shaft 302. In this example, the pair of jaws 308, 310 comprise a movable jaw 308 which is pivotal around a laterally extending pin 306 in the collar 304 to enable a gap between opposing inner surfaces of the jaws 308, 310 to be opened and closed. Although there is only one movable jaw in this example, in other embodiments, both jaws may be arranged to pivot relative to the collar 304. The collar 304 may be arranged to ensure that the jaws remain laterally aligned as they are moved together.

In the example shown in Fig. 9, the pair of jaws 308, 310 comprises a static jaw 310 that has an energy delivery structure 312 on its top surface, i.e. the surface that opposes a corresponding surface on the movable jaw 308. In use, the distal end assembly 308 is intended to grip biological tissues (and in particular a blood vessel) between the pair of jaws 308, 310. The pair of jaws 308, 310 are arranged to apply pressure to the biological tissue between the opposed surfaces and deliver energy (preferably microwave electromagnetic energy) into the tissue from the energy delivery structure 312.

In this embodiment, the energy delivery structure is present only on the static jaw 310. However, in other arrangements, there may be an energy delivery structure on both jaws, or only on a single movable jaw.

In this example, the energy delivery structure 312 comprises a coplanar microstrip antenna fabricated in the top surface of the status jaw 310. The coplanar microstrip antenna comprises a substrate 320 made of nonconductive dielectric material, e.g. ceramic or the like. The dielectric substrate 320 has a conductive layer fabricated on its underside (not visible in Fig. 9). On its top surface (i.e. the surface opposite the underside) the dielectric substrate 320 has a first conductive region in the form of a longitudinally extending finger electrode 314 disposed centrally thereon. A U-shaped second conductive region 316 is disposed on the top surface of the dielectric substrate 320 around the finger electrode 314 with a gap of exposed dielectric 315 separating the finger electrode 314 from the U-shaped region 316. A plurality of through holes 318 are formed, e.g. machined, through the U-shaped region 316 and dielectric substrate 315. The through holes 318 are filled with conductive material to electrically connect the conductive layer on the underside of the dielectric substrate 320 with the U-shaped conductive region 316. The finger electrode 314 has a contact pad 317 at a proximal end thereof. The inner conductor of the coaxial cable conveyed by the instrument shaft 302 is electrically coupled to the contact pad 317, e.g. by extending from the instrument shaft 302 to physically contact the contact pad 317. The finger electrode 314 provides an active region for the coplanar microstrip antenna. The conductive layer on the underside of the dielectric substrate 320 is electrically connected to an outer conductor of the coaxial cable conveyed by the instrument shaft 302. In conjunction with the conductive communication through the through holes 318, the U-shaped conductive region 310 forms a ground electrode for the coplanar microstrip antenna.

The configuration of the coplanar microstrip antenna shown in Fig. 9 is particularly advantageous because it confines the emitted field within the region defined by the pair of jaws 308, 310. As discussed below, very little energy is delivered to a region outside the pair of opposing surfaces. Moreover, by arranging the U-shaped conductive region 316 to extend around a distal end of the finger electrode 314, the coplanar microstrip antenna structure can prevent energy from escaping in the longitudinal direction distal to assembly 300.

The conductive layers mentioned above may be made from any suitable conductive material. Silver and gold are preferred because of their high conductivity and biocompatibility. Copper may also be used, although it is preferably plated with silver or gold in regions likely to contact biological tissue.

The coplanar microstrip antenna structure may be fabricated independently of the static jaw 310, e.g. using thin film deposition techniques. This construction of the coplanar microstrip antenna ensures two important performance features. Firstly, it ensures that the projected energy applied to the biological tissue of the gripped vessel is focused inwardly within the grasp of the instrument jaws. This provides a localised energy delivery effect, whereby the applied energy is efficiently delivered to a desired region of tissue.

Moreover, the use of thin film conductive layers means that the thermal mass of the conductive lines is minimal. In combination with the effective thermal barrier provided by the dielectric substrate 320, this means that any residual heat within the conductive lines quickly dissipates. The effect can be further enhanced by providing a layer on the surface opposing the coplanar microstrip antenna that also acts as a thermal barrier. In the embodiments shown in Fig. 9, the moveable jaw 308 has a layer of resiliently deformable material 322 formed on its inner surface. The layer 322 may be formed from silicone rubber or other compliant polymer material that can withstand the temperatures that occur during treatment and are biocompatible. They may be fabricated from an elastomeric thermoplastic polymer, for example. This layer both assists in efficient delivery of energy to gripped biological tissue, but also facilitates retaining the biological tissue within the jaws.

Alternatively or additionally, a coating may be applied to the surface of the coplanar microstrip antenna itself. This may be a coating applied only to the conductive regions, e.g. to minimise tissue sticking. In embodiments arranged to deliver microwave energy, it may not be necessary for the inner surfaces of the jaws to make direct electrical conductive contact with tissue. Accordingly, the coating may be a thin high temperature polymeric material, e.g. applied across the whole face of the antenna. The specific material may be chosen to exhibit high loss and appear transparent to the microwave energy.

The coating may conform to the shape of the jaws. It may comprise a silicone-based passivation material similar to that used as a protective coating on printed circuit boards. Other examples include polyimide, PTFE or FEP type materials.

As shown in Fig. 9, the layer 322 has a plurality of ridges moulded into it. It therefore presents a textured or toothed surface with which to contact biological tissue. A similar ridged or textured grip may be provided around the periphery of the coplanar microstrip antenna. As mentioned above, these textured surfaces can aid the release of gas during the vessel sealing operation.

The coplanar microstrip antenna has a size suitable for receiving and sealing biological vessels. For example, the coplanar microstrip antenna may be arranged to provide an effective treatment area having a width (i.e. dimension extending laterally with respect to the axis of the coaxial cable) of 2 to 5 mm and a length (along the axis of the device) of 15 to 26 mm.

Operation of the instrument 300 may be controlled by an actuation mechanism (e.g. the trigger 110 of Fig. 7), which may take the form of a scissor-type handle, slider, rotatable dial, level, trigger or the like. The actuation mechanism can be operably coupled to the instrument 300 via one or more control wires that extend along the instrument shaft 302, e.g. within the instrument channel of a scoping device. In one example, the actuation mechanism may include a force limiter arranged to limit the maximum actuation force that can be supplied to the instrument. Limiting the maximum actuation force may assist in preventing damage to delicate components in the instrument 300, and can ensure that the force applied to tissue remains within desired parameters. The force limited may comprise a compression spring or ratchet mechanism as part of the actuation mechanism. In some examples it may be desirable to vary the maximum actuation force, e.g. by provide a dial or switch on the interface joint 106 that adjusts the maximum actuation force associated with the actuation mechanism.

The pair of jaws may include a stand-off (not shown) that ensures that the jaws remain separated by a minimum distance irrespective of the closure force applied by an associated actuation mechanism located at the proximal end of the instrument shaft 302. The stand-off may be a physical projection on one or both jaws that engages the inner surface of the opposite jaw.

It is desirable for the pressure applied by jaws to tissue held therebetween to be uniform in a longitudinal direction along the inner surfaces of the jaws. In a development of the structure shown in Fig. 9, the movable jaw 308 may comprise an engagement plate at its inner surface that is capable of articulating back into the jaw 308 about a pivot point located at a distal end of the jaw 308. A resiliently deformable support element may be mounted in the jaw 308 behind the engagement plate to urge it outwardly. With this arrangement, tissue in the region between the jaws is grasped between the inner surface of the static jaw and the engagement plate of the movable jaw. As the jaws are closed, the pressure applied along the jaws is generated by a combination of the pivoting action of the jaws and the articulation of the engagement plate. The location of the pivot point and properties of the resiliently deformable support element can be selected so that the non-uniformity in applied force that arises changing mechanical advantage along the jaws away from the pivot is balanced by a cooperating non-uniformity arising from the pivotable articulation of the engagement plate.

The energy delivery structure 312 described with respect to Fig. 9 is a coplanar microstrip antenna. The configuration of that antenna may be as shown in Fig. 9, however, alterative microwave radiator structures can be used. For example the top surface of the static jaw 310 may be provided with other microstrip-based energy delivery configurations, e.g. meandering or interdigitated microstrip lines. In another embodiment, the energy delivery structure may be a travelling wave antenna.

In addition to the function of the vessel sealing, the electrosurgical instrument of the present invention also functions as a vessel divider, e.g. to cut through and separate a sealed section of a blood vessel. In one embodiment, the vessel sealer may be provided with a blade 326 that is slidably mounted with respect to the pair of jaws 308, 310 to cut through biological tissue held between the jaws. In Fig. 9, the blade 326 is shown as protruding into the region between the open jaws in Fig. 9. However, in practice, it is desirable for the instrument to prevent forward movement of the blade until after the jaws are closed and microwave energy is applied.

In the embodiment shown in Fig. 9, the blade 326 is movable in a longitudinal direction, e.g. along the axis of the device. The opposed surfaces of the jaws 308, 310 contain respective recess or guide grooves 328, 324 for receiving the blade as it travels. The guide groove 324 in the static jaw 310 is formed within the finger electrode 314 so that it moves through the centre of the applied field.

In other embodiments, the blade may be mounted within one of the jaws and arranged to move laterally with respect to the longitudinal direction, i.e. to extend out of one of the opposed surfaces into gripped tissue. The sharp edge of the blade may lie below the opposed surface during the vessel gripping and sealing operation.

In one embodiment, the cutting functionality of the blade is provided or enhanced by a magnetostrictive ultrasound transducer (not visible in Fig. 9). The transducer may have the same construction as the transducer 800 of Fig. 6A. The location of the transducer may vary between embodiments, but generally speaking it is located towards a proximal end of the blade 326 so that when the transducer generates ultrasonic vibrations, those vibrations travel along the blade 326 to enable or assist in the cutting action of the blade 326. In an embodiment, the transducer is connected to a proximal end portion of the blade 326. Where the electric drive signal for the transducer is conveyed by the coaxial cable in the instrument shaft 302, the transducer has a first input terminal coupled to one conductor (e.g. inner conductor) of the coaxial cable and a second input terminal coupled to the other conductor (e.g. outer conductor) of the coaxial cable. For example, first and second connectors (e.g. wires, tracks, cables, and conductors) may couple each terminal to its respective conductor of the coaxial cable. In this way, an electrical (e.g. current) signal for driving the magnetostrictive ultrasound transducer may be conveyed by the coaxial cable, and delivered to the transducer at the proximal end of the blade 326. In operation, the transducer may generate ultrasonic vibrations which enable or enhance a cutting action of the blade 326. Specifically, as described above with reference to Figs. 1 to 5, the electrical signal may oscillate at an ultrasound frequency. On receiving this electrical signal, the coil of the transducer induces an oscillating magnetic field around the magnetostrictive element the transducer such that the magnetostrictive effect causes the magnetostrictive element to rapidly expand and contract at the ultrasonic frequency thereby generating ultrasonic vibrations. Since the transducer is coupled to the blade 326, these ultrasonic vibrations travel into the blade 326 and are then radiated into the tissue surrounding the blade 326. The vibrations produce mechanical friction that generates thermal energy thereby resulting in extracellular heating followed subsequently by intracellular heating. In this way, the ultrasound vibrations can be used to treat (i.e. cut) the biological tissue.

The distal end assembly may be configured to perform functions in addition to vessel sealing. For example, the distal end assembly may have an auxiliary radiofrequency (RF) cutting blade mounted on a distal tip thereon. In the example shown in Fig. 9, an RF dissector element 330 is mounted on the distal end of the static jaw 310. The RF dissector element 330 is a bipolar structure that comprises an active electrode mounted on a protruding body, and a return electrode, which may be fabricated on or integrated with the static jaw 310 in the vicinity of the protruding body.

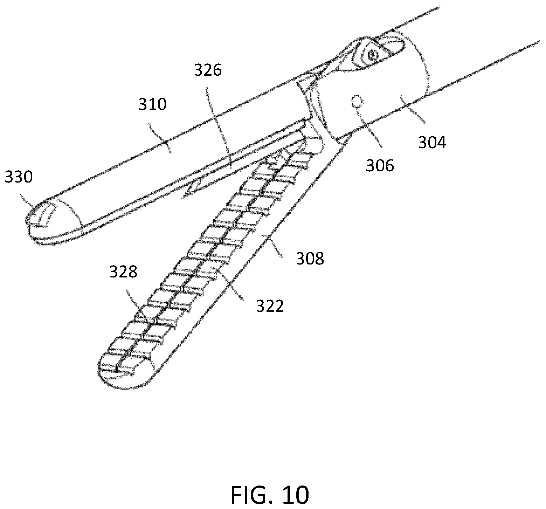

Fig. 10 shows the underside of the distal end assembly 300, where the RF dissector element 330 can be seen in more detail. The RF dissector element 330 can be used for fine bloodless tissue cutting and tissue dissection. In the arrangement shown in Figs. 9 and 10, the RF dissector element 330 presents a leading edge that sits proud of the distal end of the static jaw 310 This position can enable both side and end-on dissection to be performed. In dry field treatment scenarios (i.e. in the absence of saline or other electrically conductive fluid) it is desirable for the return electrode to be in close proximity to the active electrode that is on the RF dissector element 330. The ratio of the exposed tissue contacting electrode areas is also important to ensure that current flow occurs in a desired manner that causes maximum current density to occur on the leading edge of the RF dissector element 330.

Although the RF dissector element 330 is shown at the distal end of the static jaw in Figs. 9 and 10, it can be mounted in a variety of orientations or locations on the distal end assembly, e.g. vertically, horizontally, at an angle, on one side, and on either jaw.

The pair of jaws may have any suitable shape. For example, the jaws may be tapered along their length towards the distal tip, or may be bent or hooked if desired for any particular treatment scenario.

Opening and closing of the jaws 308, 310 may be controlled by an actuation mechanism that is operable by a user at an external handle of the surgical scoping device, i.e. at a proximal end of the instrument shaft 302 (e.g. trigger 110 of interface joint 106). The actuation mechanism may include a pressure control device arranged to enable a user to control closure of the pair of jaws based on an amount of pressure applied to the biological tissue that is captured between the jaws. In one example, a user may select a desired (e.g. maximum) closure pressure for the jaws, and the actuation mechanism may be arranged to inhibit further movement of the jaws towards each other once the desired pressure is reached.

As mentioned above, in some embodiments, both of the jaws may be active in the sense that they are electrically connected to a coaxial cable within the instrument shaft. In one example the pair of jaws comprise different elements of a single microwave energy delivery device. For example, one of the jaws may comprise a ground electrode, and the other may comprise an active electrode for an antenna structure. In another example, each jaw may comprise its own independent microwave energy delivery structure, e.g. corresponding to the coplanar microstrip antenna described above.

If both of the jaws are active, they may be fed from a common coaxial transmission line within the instrument shaft by providing a microwave power divider or splitter at the distal end of the coaxial transmission line, e.g. at the distal end of the instrument shaft, or within the collar 304. The microwave power splitter may be implemented in any known manner. For example, the power splitter could be implemented as a Wilkinson power splitter, as two quarter wavelength (or odd multiple thereof) impedance transformers or as a half wavelength balun arrangement, where the distal end of the coaxial line forms an unbalanced feed that is input to the first jaw, and where the second jaw is fed from a point that is half an electrical wavelength away from the feed. Alternatively, the power splitter may be implemented as half electrical wavelength impedance transformers that are fabricated using flexible substrate materials, which are able to flex to allow for moving one or both jaws.

In arrangements where the distal end assembly also includes an auxiliary device for delivering RF energy, the instrument may be arranged to receive the RF energy for the auxiliary device and the microwave energy for delivery from the jaws along a common energy delivery pathway, which may be a coaxial transmission line within the instrument shaft. In one example, RF energy may be delivered at 400 kHz, whereas the microwave energy may be delivered at 5.8 GHz. In order to prevent the microwave energy from entering the auxiliary device an inductive blocking or filtering component may be mounted within the distal end assembly. The inductive block may be a wire-wound inductor, which permits RF energy to pass through the use of parasitic effects, but blocks microwave energy. Alternatively, the inductive block may be provided by one or more quarter wavelength open stubs located at half wavelength intervals along a transmission line between the coaxial cable and the auxiliary RF device. In order to prevent RF energy from entering the microwave energy delivery structure in the jaws, a capacitive block or filter element may be mounted between the coaxial cable and the microwave energy delivery structure. The capacitive filter element may be a parallel plate capacitor that operates at microwave frequencies, or a waveguide cavity or coupled microstrip line where an insulating dielectric breaks the conductive path in the manner that blocks flow of RF energy.

Similar blocks or filters may be used at the generator to prevent RF energy from entering the microwave source and microwave energy from entering the RF source. For example one or more chokes may be provided to prevent microwave energy from radiating into the RF source.

In the example above, the RF and microwave energy is carried along the instrument shaft by a common coaxial transmission line. In other examples, the separation of the RF and microwave energy may occur before they are delivered into the instrument shaft. In this arrangement, separate energy conveying structures are provided for the RF energy and microwave energy respectively. For example, the RF energy can be conveyed by a twisted wire pair or two insulated wire assemblies mounted in parallel, whilst the microwave energy is carried by a suitable coaxial transmission line. Power for the ultrasound transducer can be delivered in a similar manner, e.g. in a coaxial cable with the microwave EM energy (and RF EM energy), or separately from the coaxial cable in a twisted wire pair (e.g. separately or with the RF EM energy).

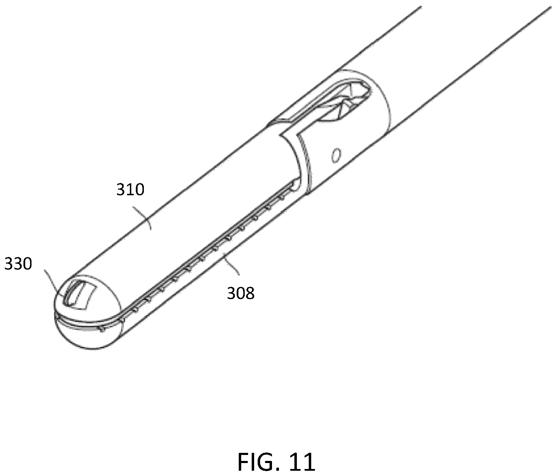

Fig. 11 shows a view of the underside of the distal end assembly when the jaws 308, 310 are closed. This is a configuration in which the instrument may be introduced to an instrument channel of a laparoscope.

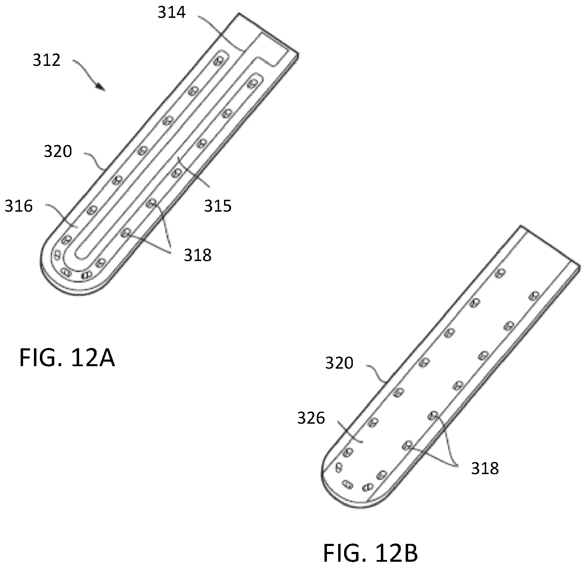

Figs. 12A and 12B show in more detail a first example of a coplanar microstrip antenna that can be used as an energy delivery structure 312 in an embodiment of the invention. The coplanar microstrip antenna comprises a dielectric substrate 320 which has a conductive ground layer 336 on its under surface (see Fig. 12B) and a pair of conductor lines 314, 316 on its upper surface. The ground layer 336 and the conductor lines 314, 316 may be formed on the substrate using any suitable technique, e.g. metallisation, thin film deposition and patterning (etching), etc.

As discussed above, the pair of conductive lines 314, 316 in this example comprise a finger electrode 314 that is surrounded along its length and around its distal end by a U-shaped conductive region 316. The U-shaped conductive region 316 is electrically connected to the ground layer 336 via through holes 318, 338 which are filled with conductive material to provide an electrical connection. The finger electrode 314 and u-shaped conductive region 316 are separated by a gap 315 across which the microwave field is concentrated in use. The ground conductor 336 is in electrical communication with an outer conductor of a coaxial feedline, whereas the finger electrode 314 is electrically connected to an inner conductor of the coaxial feedline.

In a variant of the embodiment of Figs. 9 to 12B, the blade 326 or blade mechanism may not include a magnetostrictive ultrasound transducer and, instead, the blade 326 may provide a "cold" cut, and comprise a sharp scalpel-type structure, made of steel or other hard material. However, cutting functionality could additionally or alternatively be provided or enhanced by thermal means other than ultrasonic vibration, e.g. a radiofrequency (RF) monopolar or bipolar energy delivery structure. An arrangement for delivering auxiliary power down the instrument shaft, e.g. for an RF cutting blade, is discussed above. In such cases, the magnetostrictive ultrasound transducer may be located elsewhere in the distal tip assembly. Three different embodiments will now be described.

Firstly, the magnetostrictive ultrasound transducer may be located in or on one of the jaws. Where the transducer is "in" one of the jaws, the transducer may be completely inside the jaw's volume, and where the transducer is "on" one of the jaws, the transducer may be on a surface of the jaw and, possibly, partly inside the jaw's volume. As such, when the transducer generates ultrasonic vibrations the whole jaw vibrates to assist in treating (e.g. dividing) tissue positioned between the jaws. In the case where the jaw 308 does not include a microwave or RF delivery structure, the jaw 308 may contain the transducer encased (partly or completely) within its volume. As such, the jaw 310 may function to deliver microwave EM energy into tissue positioned between the jaws, and the jaw 308 may function to deliver ultrasonic vibrations into tissue positioned between the jaws. It is to be understood, that in this embodiment the transducer is electrically coupled to the structure (e.g. coaxial cable, twisted wire pair) for conveying the transducer's electrical drive signal through the instrument shaft 302.

Secondly, in another embodiment in which one of the jaws includes the magnetostrictive ultrasound transducer for delivering ultrasonic energy to perform cutting, the transducer may be mounted on an independently slidable member that can be longitudinally extended and retracted with respect to the instrument shaft 302. This can assist in improving visibility of fine treatment using the transducer, as it can be extended into the field of view of the surgical scoping device independently of the rest of the distal end assembly 300. In one embodiment, the independently slidable member may be the static jaw 310, which can be dislocated from the collar 304 to enable it to slide longitudinally. The static jaw may be either retractable proximally away from its normal hinged location, or may be extendable distally away from its normal hinged location. In the latter scenario, the transducer may be located on the static jaw, so that it is can be moved into a distalmost position. In the former scenario, transducer may be located on the opposing jaw so that is occupies a distalmost position having good visibility when the static jaw is retracted.

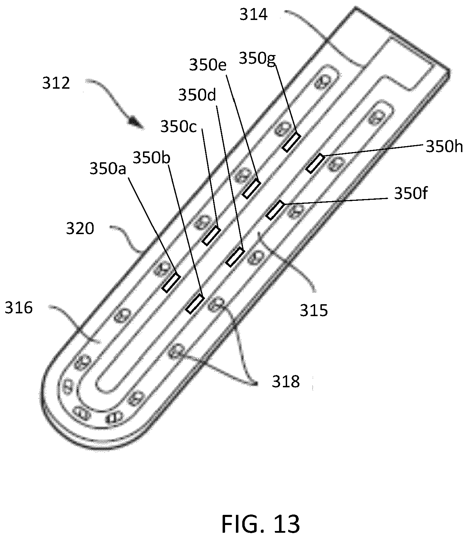

Thirdly, in a further embodiment, a single jaw (e.g. jaw 310) may deliver both microwave EM energy and ultrasonic vibrations into tissue positioned in-between the jaws. For example, considering the coplanar microwave antenna structure of Figs. 12A and 12B, one or more magnetostrictive ultrasound transducers may be positioned within the coplanar microwave structure, e.g. in-between the active conductive strip and the ground conductive strip. For example, the one or more transducers may be arranged in-between the finger electrode 314 and the U-shaped electrode 316. Since space is restricted in this location, it may be necessary to include multiple transducers because the maximum size for each individual transducer may be smaller compared to the aforementioned embodiments in which the transducer positioned either at the proximal end of the blade 326 or in/on one of the jaws. Therefore, the combined magnitude of the ultrasonic vibrations generated by multiple smaller transducers positioned in-between the electrodes 314 and 316 may be comparable to the magnitude of the ultrasonic vibrations generated by a single larger transducer positioned either at the proximal end of the blade 326 or in/on one of the jaws. Furthermore, each transducer positioned in-between the electrodes 314 and 316 may be located at or near a top surface of the dielectric substrate 320 such that the source of the ultrasonic vibrations is as close as possible to the tissue being treated, in order to minimise attenuation of the vibrations before they reach the tissue. In an embodiment, two, three, five, ten or more transducers may be positioned in-between the electrodes 314 and 316. An embodiment with eight such transducers 350a-h is shown in Fig. 13. Though connection lines are not visible in Fig. 13, it is to be understood that each transducer is electrically coupled to the structure for conveying the transducer's electrical drive signal through the instrument shaft 302 (e.g. coaxial cable, twisted wire pair). For example, each transducer 350a-h may have a first terminal coupled to ground layer 336 (e.g. via electrode 316) and a second terminal coupled to the electrode 314. Also, each transducer 350a-h may be driven by the same or separate electrical drive signals.

In the aforementioned description with respect to Figs. 8 to 13, various embodiments have been described in which one or more magnetostrictive ultrasound transducers are positioned on a distal end assembly of an electrosurgical instrument. However, in a further embodiment, the distal end assembly may not include any magnetostrictive ultrasound transducers and, instead, the instrument shaft (e.g. a distal end) may include one or more magnetostrictive ultrasound transducers. In this way, the or each transducer can be easily coupled to the structure within the shaft which conveys the electrical signal for driving the or each transducer. Also, the ultrasonic vibrations generated by the or each transducer are generated at the distal end of the shaft can travel through the distal end assembly of the instrument to reach biological tissue for tissue treatment. An advantage of this arrangement is that finding a suitable space for housing the transducer and providing connections between the shaft and transducer may be easier.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention

For the avoidance of any doubt, any theoretical explanations provided herein are provided for the purposes of improving the understanding of a reader. The inventors do not wish to be bound by any of these theoretical explanations.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An electrosurgical generator comprising:

    an electromagnetic (EM) signal supply unit for generating EM energy;
    an electrical signal supply unit for generating an electrical signal for driving a magnetostrictive ultrasound transducer;
    an output port configured to be connectable to an electrosurgical instrument for delivering the EM energy from a distal end thereof and for generating ultrasonic vibrations using the electrical signal; and
    a feed structure for conveying the EM energy from the EM signal supply unit to the output port, and for conveying the electrical signal from the electrical signal supply unit to the output port, wherein the feed structure has a common signal pathway for conveying the EM energy and the electrical signal to the output port wherein the electrical signal supply unit comprises:

       a first power supply for outputting a first supply signal and a second supply signal;
       a signal source for outputting a first control signal and a second control signal;
       a first switching circuit having a control input coupled to the signal source for receiving the first control signal, a supply input coupled to the first power supply for receiving the first supply signal, and an output, wherein the first switching circuit is operable to provide at the output at least part of the electrical signal based on the first supply signal and the first control signal;
       a second switching circuit having a control input coupled to the signal source for receiving the second control signal, a supply input coupled to the second power supply for receiving the second supply signal, and an output, wherein the second switching circuit is operable to provide at the output a second part of the electrical signal based on the second supply signal and the second control signal, and
       a common signal pathway coupled the output of the first switching circuit for receiving a first part of the electrical signal, and coupled to the output of the second switching circuit for receiving the second part of the electrical signal, and for combining the first and second parts to form the electrical signal.

2. The electrosurgical generator of claim 1 wherein the EM signal supply unit comprises a microwave signal generator for generating microwave EM radiation having a first frequency, and

    wherein the feed structure comprises an electrical signal channel for connecting the output port to the electrical signal supply unit, and a microwave channel for connecting the output port to the microwave signal generator, the electrical signal channel and microwave channel comprising physically separate signal pathways from the electrical signal supply unit and microwave signal generator respectively,
    wherein the feed structure includes a first combining circuit having a first input connected to receive the electrical signal from the electrical signal channel, a second input connected to receive the microwave EM radiation from the microwave channel, and an output in communication with the first and second inputs for transferring the electrical signal and the microwave EM radiation to the common signal pathway.

3. The electrosurgical generator of claim 2, wherein the microwave channel comprises a first filter arranged to permit the passage of microwave EM radiation from the microwave signal generator to the first combining circuit, but prevent the passage of the electrical signal from the first combining circuit to the microwave signal generator.

4. The electrosurgical generator of claim 1 or 2, wherein the electrical signal channel comprises a second filter arranged to permit the passage of the electrical signal from the electrical signal supply unit to the first combining circuit, but prevent the passage of the microwave EM radiation from the first combining circuit to the electrical signal supply unit.

5. The electrosurgical generator of any one of claims 1 to 4, wherein the electromagnetic signal supply unit comprises:

a radiofrequency (RF) signal generator for generating RF EM radiation having a second frequency that is lower than the first frequency, wherein the feed structure comprises an RF channel for connecting the output port to the RF signal generator, the RF channel and microwave channel comprising physically separate signal pathways from the RF signal generator and microwave signal generator respectively, wherein the feed structure includes a second combining circuit connected to the electrical signal channel and having a first input connected to receive the electrical signal from the electrical signal supply unit and a second input connected to receive the RF EM radiation from the RF channel, and an output in communication with the first and second inputs for transferring the RF EM radiation and the electrical signal to the first combining circuit.

6. The electrosurgical generator of claim 5, wherein the electrical signal channel comprises a third filter arranged to permit the passage of the electrical signal from the electrical signal supply unit to the second combining circuit, but prevent the passage of the RF EM radiation from the second combining circuit to the electrical signal supply unit.

7. The electrosurgical generator of claim 5 or 6, wherein the RF channel comprises a fourth filter arranged to permit the passage of the RF EM radiation from the RF signal generator to the second combining circuit, but prevent the passage of the electrical signal from the second combining circuit to the RF signal generator.

8. The electrosurgical generator of claim 1, wherein the first power supply comprises a filtering circuit for blocking an alternating current signal received by the first power supply at its output.

9. The electrosurgical generator of any one of claims 1 to 8, wherein the first switching circuit comprises a current source for generating the at least part of the electrical signal from the first supply signal and in accordance with a characteristic of the first control signal.

10. The electrosurgical generator of any one of claims 1 to 9, wherein the first switching circuit comprises a signal conditioner coupled to a switch, the signal conditioner being operable to convert the control signal into a driving signal for operating the switch.

11. An electrosurgical apparatus comprising:

an electrosurgical instrument for delivering electromagnetic (EM) energy and ultrasonic vibrations for treating biological tissue, the electrosurgical instrument comprising:

an instrument shaft arranged to convey EM energy and an electrical signal for driving a magnetostrictive ultrasound transducer; a distal end assembly arranged at a distal end of the instrument shaft to receive the EM energy from the instrument shaft and deliver the EM energy from the distal end assembly for tissue treatment; and a magnetostrictive ultrasound transducer arranged to receive the electrical signal from the instrument shaft and generate ultrasonic vibrations around the distal end assembly for tissue treatment, and

an electrosurgical generator as claimed in any of claims 1 to 10, wherein the output of the electrosurgical generator is configured to be connectable to a proximal end of the instrument shaft of the electrosurgical instrument.

**Patentansprüche**

1. Elektrochirurgischer Generator, umfassend:

eine Elektromagnetisches- (EM-) Signal-Zufuhreinheit zum Erzeugen von EM-Energie; eine Elektrisches-Signal-Zufuhreinheit zum Erzeugen eines elektrischen Signals zum Ansteuern eines magnetostriktiven Ultraschallwandlers; einen Ausgangsanschluss, der konfiguriert ist, um mit einem elektrochirurgischen Instrument zum Zuführen der EM-Energie von einem distalen Ende davon und zum Erzeugen von Ultraschallschwingungen unter Verwendung des elektrischen Signals verbindbar zu sein; und eine Zufuhrstruktur zum Übertragen der EM-Energie von der EM-Signaleinheit an den Ausgangsanschluss und zum Übertragen des elektrischen Signals von der Elektrisches-Signal-Zufuhreinheit an den Ausgangsanschluss, wobei die Zufuhrstruktur einen gemeinsamen Signalpfad zum Übertragen der EM-Energie und des elektrischen Signals an den Ausgangsanschluss aufweist, wobei

die Elektrisches-Signal-Zufuhreinheit Folgendes umfasst:

eine erste Leistungsversorgung zum Ausgeben eines ersten Zufuhrsignals und eines zweiten Zufuhrsignals;

eine Signalquelle zum Ausgeben eines ersten Steuersignals und eines zweiten Steuersignals;

einen ersten Schaltschaltkreis, der einen Steuereingang, der mit der Signalquelle zum Empfangen des ersten Steuersignals gekoppelt ist, einen Zufuhreingang, der mit der ersten Leistungsversorgung zum Empfangen des ersten Zufuhrsignals gekoppelt ist, und einen Ausgang aufweist, wobei der erste Schaltschaltkreis betreibbar ist, um an dem Ausgang zumindest einen Teil des elektrischen Signals basierend auf dem ersten Zufuhrsignal und dem ersten Steuersignal bereitzustellen;

einen zweiten Schaltschaltkreis, der einen Steuereingang, der mit der Signalquelle zum Empfangen des zweiten Steuersignals gekoppelt ist, einen Zufuhreingang, der mit der zweiten Leistungsversorgung zum Empfangen des zweiten Zufuhrsignals gekoppelt ist, und einen Ausgang aufweist, wobei der zweite Schaltschaltkreis betreibbar ist, um an dem Ausgang einen zweiten Teil des elektrischen Signals basierend auf dem zweiten Zufuhrsignal und dem zweiten Steuersignal bereitzustellen, und

einen gemeinsamen Signalpfad, der mit dem Ausgang des ersten Schaltschaltkreises zum Empfangen eines ersten Teils des elektrischen Signals gekoppelt ist, und der mit dem Ausgang des zweiten Schaltschaltkreises zum Empfangen des zweiten Teils des elektrischen Signals und zum Kombinieren des ersten und des zweiten Teils zum Bilden des elektrischen Signals gekoppelt ist.

2. Elektrochirurgischer Generator nach Anspruch 1, wobei die EM-Signalzufuhreinheit einen Mikrowellensignalgenerator zum Erzeugen von EM-Mikrowellenstrahlung mit einer ersten Frequenz umfasst und

wobei die Zufuhrstruktur einen Elektrisches-Signal-Kanal zum Verbinden des Ausgangsanschlusses mit der Elektrisches-Signal-Zufuhreinheit und einen Mikrowellenkanal zum Verbinden des Ausgangsanschlusses mit dem Mikrowellensignalgenerator umfasst, wobei der Elektrisches-Signal-Kanal und der Mikrowellenkanal physisch getrennte Signalwege von der Elektrisches-Signal-Zufuhreinheit bzw. dem Mikrowellensignalgenerator umfassen,

wobei die Zufuhrstruktur eine erste Kombinationsschaltung mit einem ersten Eingang, der verbunden ist, um das elektrische Signal von dem Elektrisches-Signal-Kanal zu empfangen, einen zweiten Eingang, der verbunden ist, um die EM-Mikrowellenstrahlung von dem Mikrowellenkanal zu empfangen, und einen Ausgang in Kommunikation mit dem ersten und dem zweiten Eingang zum Übertragen des elektrischen Signals und der EM-Mikrowellenstrahlung an den gemeinsamen Signalpfad aufweist.

3. Elektrochirurgischer Generator nach Anspruch 2, wobei der Mikrowellenkanal einen ersten Filter umfasst, der angeordnet ist, um den Durchtritt von EM-Mikrowellenstrahlung von dem Mikrowellensignalgenerator zur ersten Kombinationsschaltung zuzulassen, aber den Durchtritt des elektrischen Signals von der ersten Kombinationsschaltung zum Mikrowellensignalgenerator zu verhindern.

4. Elektrochirurgischer Generator nach Anspruch 1 oder 2, wobei der Elektrisches-Signal-Kanal einen zweiten Filter umfasst, der angeordnet ist, um den Durchtritt des elektrischen Signals von der Elektrisches-Signal-Zufuhreinheit zur ersten Kombinationsschaltung zuzulassen, aber den Durchtritt der EM-Mikrowellenstrahlung von der ersten Kombinationsschaltung zur Elektrisches-Signal-Zufuhreinheit zu verhindern.

5. Elektrochirurgischer Generator nach einem der Ansprüche 1 bis 4, wobei die Elektromagnetisches-Signal-Zufuhreinheit Folgendes umfasst:

einen Hochfrequenz- (HF-) Signalgenerator zum Erzeugen von EM-HF-Strahlung mit einer zweiten Frequenz, die niedriger ist als die erste Frequenz,

wobei die Zufuhrstruktur einen HF-Kanal zum Verbinden des Ausgangsanschlusses mit dem HF-Signalgenerator umfasst, wobei der HF-Kanal und der Mikrowellenkanal physisch getrennte Signalwege von dem HF-Signalgenerator bzw. dem Mikrowellensignalgenerator umfassen,

wobei die Zufuhrstruktur eine zweite Kombinationsschaltung umfasst, die mit dem Elektrisches-Signal-Kanal verbunden ist, und die einen ersten Eingang, der verbunden ist, um das elektrische Signal von der Elektrisches-Signal-Zufuhreinheit zu empfangen, und einen zweiten Eingang, der verbunden ist, um die EM-HF-Strahlung von dem HF-Kanal zu empfangen, und einen Ausgang in Kommunikation mit dem ersten und dem zweiten Eingang zum Übertragen der EM-HF-Strahlung und des elektrischen Signals an die erste Kombinationsschaltung aufweist.

6. Elektrochirurgischer Generator nach Anspruch 5, wobei der elektrisches-Signal-Kanal einen dritten Filter umfasst, der angeordnet ist, um den Durchtritt des elektrischen Signals von der Elektrisches-Signal-Zufuhreinheit zur zweiten Kombinationsschaltung zuzulassen, aber den Durchtritt der EM-HF-Strahlung von der zweiten Kombinationsschaltung zur Elektrisches-Signal-Zufuhreinheit zu verhindern.

7. Elektrochirurgischer Generator nach Anspruch 5 oder 6, wobei der HF-Kanal einen vierten Filter umfasst, der angeordnet ist, um den Durchtritt der EM-HF-Strahlung von dem HF-Signalgenerator zur zweiten Kombinationsschaltung zuzulassen, aber den Durchtritt des elektrischen Signals von der zweiten Kombinationsschaltung zum HF-Signalgenerator zu verhindern.

8. Elektrochirurgischer Generator nach Anspruch 1, wobei die erste Leistungsversorgung eine Filterschaltung zum Blockieren eines Wechselstromsignals, das von der ersten Leistungsversorgung an deren Ausgang empfangen wird, umfasst.

9. Elektrochirurgischer Generator nach einem der Ansprüche 1 bis 8, wobei der erste Schaltschaltkreis eine Stromquelle zum Erzeugen des zumindest einen Teils des elektrischen Signals von dem ersten Zufuhrsignal und gemäß einer Eigenschaft des ersten Steuersignals umfasst.

10. Elektrochirurgischer Generator nach einem der Ansprüche 1 bis 9, wobei der erste Schaltschaltkreis einen Signalkonditionierer umfasst, der mit einem Schalter gekoppelt ist, wobei der Signalkonditionierer betreibbar ist, um das Steuersignal in ein Ansteuersignal zum Betreiben des Schalters umzuwandeln.

11. Elektrochirurgische Vorrichtung, umfassend:

ein elektrochirurgisches Instrument zum Zuführen von elektromagnetischer (EM-) Energie und von Ultraschallschwingungen zur Behandlung von biologischem Gewebe, wobei das elektrochirurgische Instrument Folgendes umfasst:

einen Instrumentschaft, der angeordnet ist, um EM-Energie und ein elektrisches Signal zum Ansteuern eines magnetostriktiven Ultraschallwandlers zu übermitteln; eine distale Endanordnung, die an einem distalen Ende des Instrumentschafts angeordnet ist, um die EM-Energie von dem Instrumentschaft zu empfangen und die EM-Energie von der distalen Endanordnung zur Gewebebehandlung zuzuführen; und einen magnetostriktiven Ultraschallwandler, der angeordnet ist, um das elektrische Signal von dem Instrumentschaft zu empfangen und Ultraschallschwingungen rund um die distale Endanordnung zur Gewebebehandlung zu erzeugen, und

einen elektrochirurgischen Generator nach einem der Ansprüche 1 bis 10, wobei der Ausgang des elektrochirurgischen Generators konfiguriert ist, um mit einem proximalen Ende des Instrumentschafts des elektrochirurgischen Instruments verbindbar zu sein.

## Revendications

1. Générateur électrochirurgical, comprenant :

une unité d'alimentation de signal électromagnétique (EM) pour générer de l'énergie EM ; une unité d'alimentation de signal électrique pour générer un signal électrique pour entraîner un transducteur ultrasonore magnétostrictif ; un port de sortie configuré pour pouvoir être connecté à un instrument électrochirurgical pour délivrer l'énergie EM à partir d'une extrémité distale de celui-ci et pour générer des vibrations ultrasonores en utilisant le signal électrique ; et une structure d'alimentation pour transporter l'énergie EM de l'unité d'alimentation de signal EM au port de sortie, et pour transporter le signal électrique de l'unité d'alimentation de signal électrique au port de sortie, dans lequel la structure d'alimentation présente un trajet de signal commun pour transporter l'énergie EM et le signal électrique au port de sortie, dans lequel l'unité d'alimentation de signal électrique comprend :

une première alimentation électrique pour délivrer en sortie un premier signal d'alimentation et un second signal d'alimentation ; une source de signal pour délivrer en sortie un premier signal de commande et un second signal de commande ; un premier circuit de commutation présentant une entrée de commande couplée à la source de signal pour recevoir le premier signal de commande, une entrée d'alimentation couplée à la première alimentation pour recevoir le premier signal d'alimentation, et une sortie, dans lequel le premier circuit de commutation est opérationnel pour fournir à la sortie au moins une partie du signal électrique sur la base du premier signal d'alimentation et du premier signal de commande ;

un second circuit de commutation présentant une entrée de commande couplée à la source de signal pour recevoir le second signal de commande, une entrée d'alimentation couplée à la seconde alimentation pour recevoir le second signal d'alimentation, et une sortie, dans lequel le second circuit de commutation peut fonctionner pour fournir en sortie une seconde partie du signal électrique sur la base du second signal d'alimentation et du second signal de commande, et

un trajet de signal commun couplé à la sortie du premier circuit de commutation pour recevoir une première partie du signal électrique, et couplé à la sortie du second circuit de commutation pour recevoir la seconde partie du signal électrique, et pour combiner les première et seconde parties pour former le signal électrique.

2. Générateur électrochirurgical selon la revendication 1, dans lequel l'unité d'alimentation en signal EM comprend un générateur de signal hyperfréquence pour générer un rayonnement EM hyperfréquence présentant une première fréquence, et

dans lequel la structure d'alimentation comprend un canal de signal électrique pour connecter le port de sortie à l'unité d'alimentation en signal électrique, et un canal hyperfréquence pour connecter le port de sortie au générateur de signal hyperfréquence, le canal de signal électrique et le canal hyperfréquence comprenant des trajets de signal physiquement séparés de l'unité d'alimentation en signal électrique et du générateur de signal hyperfréquence respectivement,

dans lequel la structure d'alimentation inclut un premier circuit de combinaison présentant une première entrée connectée pour recevoir le signal électrique à partir du canal de signal électrique, une seconde entrée connectée pour recevoir le rayonnement EM hyperfréquence à partir du canal hyperfréquence, et une sortie en communication avec les première et seconde entrées pour transférer le signal électrique et le rayonnement EM hyperfréquence au trajet de signal commun.

3. Générateur électrochirurgical selon la revendication 2, dans lequel le canal hyperfréquence comprend un premier filtre agencé pour permettre le passage du rayonnement électromagnétique hyperfréquence du générateur de signal hyperfréquence au premier circuit de combinaison, mais empêcher le passage du signal électrique du premier circuit de combinaison au générateur de signal hyperfréquence.

4. Générateur électrochirurgical selon la revendication 1 ou 2, dans lequel le canal de signal électrique comprend un deuxième filtre agencé pour permettre le passage du signal électrique de l'unité d'alimentation de signal électrique au premier circuit de combinaison, mais empêcher le passage du rayonnement EM hyperfréquence du premier circuit de combinaison à l'unité d'alimentation de signal électrique.

5. Générateur électrochirurgical selon l'une quelconque des revendications 1 à 4, dans lequel l'unité d'alimentation de signal électromagnétique comprend :

un générateur de signal radiofréquence (RF) pour générer un rayonnement RF EM présentant une seconde fréquence qui est inférieure à la première fréquence,

dans lequel la structure d'alimentation comprend un canal RF pour connecter le port de sortie au générateur de signal RF, le canal RF et le canal hyperfréquence comprenant des voies de signal physiquement séparées du générateur de signal RF et du générateur de signal hyperfréquence, respectivement,

dans lequel la structure d'alimentation inclut un second circuit de combinaison connecté au canal de signal électrique et présentant une première entrée connectée pour recevoir le signal électrique à partir de l'unité d'alimentation de signal électrique et une seconde entrée connectée pour recevoir le rayonnement RF EM à partir du canal RF, et une sortie en communication avec les première et seconde entrées pour transférer le rayonnement RF EM et le signal électrique vers le premier circuit de combinaison.

6. Générateur électrochirurgical selon la revendication 5, dans lequel le canal de signal électrique comprend un troisième filtre agencé pour permettre le passage du signal électrique de l'unité d'alimentation de signal électrique au second circuit de combinaison, mais empêcher le passage du rayonnement RF EM du second circuit de combinaison à l'unité d'alimentation de signal électrique.

7. Générateur électrochirurgical selon la revendication 5 ou 6, dans lequel le canal RF comprend un quatrième filtre agencé pour permettre le passage du rayonnement RF EM du générateur de signal RF au second circuit de combinaison, mais empêcher le passage du signal électrique du second circuit de combinaison au générateur de signal RF.

8. Générateur électrochirurgical selon la revendication 1, dans lequel la première alimentation électrique comprend un circuit de filtrage pour bloquer un signal de courant alternatif reçu par la première alimentation électrique au niveau de sa sortie.

9. Générateur électrochirurgical selon l'une quelconque des revendications 1 à 8, dans lequel le premier circuit de commutation comprend une source de courant pour générer la au moins une partie du signal électrique à partir du premier signal d'alimentation et conformément à une caractéristique du premier signal de commande.

10. Générateur électrochirurgical selon l'une quelconque des revendications 1 à 9, dans lequel le premier circuit de commutation comprend un conditionneur de signal couplé à un commutateur, le conditionneur de signal pouvant fonctionner pour convertir le signal de commande en un signal d'entraînement pour actionner le commutateur.

11. Appareil électrochirurgical, comprenant :

un instrument électrochirurgical pour délivrer une énergie électromagnétique (EM) et des vibrations ultrasonores afin de traiter un tissu biologique, l'instrument électrochirurgical comprenant :

une tige d'instrument agencée pour transporter de l'énergie EM et un signal électrique pour entraîner un transducteur ultrasonore magnétostrictif ;
un ensemble d'extrémité distale agencé au niveau d'une extrémité distale de la tige d'instrument pour recevoir l'énergie EM à partir de la tige d'instrument, et délivrer l'énergie EM à partir de l'ensemble d'extrémité distale pour un traitement de tissu ;
un transducteur ultrasonore magnétostrictif agencé pour recevoir le signal électrique à partir de la tige d'instrument et générer des vibrations ultrasonores autour de l'ensemble d'extrémité distale pour un traitement des tissu, et

un générateur électrochirurgical selon l'une quelconque des revendications 1 à 10,
dans lequel la sortie du générateur électrochirurgical est configurée pour pouvoir être connectée à une extrémité proximale de la tige d'instrument de l'instrument électrochirurgical.

FIG. 1

400

490

492

494

496

498

482

484

470

472

476

480

474

478

468

462

466

458

460

456  $V_{16}$

454

444

446

448  $V_4$

440  $V_{15}$

442

432

428

430

438

434

436

424

402

404  $V_{10}$

408  $V_{11}$

410

412

414

416

415

418  $V_{13}$

422  $V_{12}$

426

427

417

419

420

406

$V_{10}$ $V_{11}$ $V_{12}$ $V_{13}$ $V_{14}$ $V_{15}$ $V_{16}$

A  B  C

450

452

464

A  B  C

Electrical signal for ultrasound transducer

506.

502.

504.

508.

500.

490

FIG. 2

FIG. 3

EP 4 144 315 B1

700

706.

702.

704.

+V
0 ———→ t

+i
0 ———→ t

Electrical signal
for ultrasound
transducer

FIG. 4

FIG. 5

FIG. 6B

FIG. 6A

FIG. 7

Fig. 8

EP 4 144 315 B1

FIG. 9

FIG. 10

EP 4 144 315 B1

FIG. 11

FIG. 12A

FIG. 12B

EP 4 144 315 B1

FIG. 13

**REFERENCES CITED IN THE DESCRIPTION**

**Patent documents cited in the description**

- GB 2486343 A
- US 6585735 B
- EP 2233098 A
- WO 2015097472 A
- GB 2569812 A
- WO 2017103209 A1